# EUROPEAN PATENT APPLICATION

(11) **EP 4 789 758 A1**
(43) Date of publication of application: **12.08.2026**
(21) Application number: 25156650.1
(22) Date of filing: 07.02.2025
(51) Int. Cl.: B01J 8/22, B01J 23/44, C01B 21/14, C07C 249/08

(54) **PROCESS AND DEVICE FOR PREPARING HYDROXYLAMINE**

(71) Applicant: CAP III B.V., 6129 EL Urmond (NL)
(72) Inventor: TINGE, Johan Thomas, 6129 EL Urmond (NL); OLZHEIM, Daniel Julius Maria, 6129 EL Urmond (NL); DAGUENET, Corinne, 6129 EL Urmond (NL)
(74) Representative: Cohausz & Florack

(57) **Abstract**

The invention relates to a continuous process for preparing hydroxylamine in a buffered aqueous phosphoric acid-containing solution by hydrogenating nitrate with hydrogen at a pressure above atmospheric pressure in the presence of suspended Pd-containing hydrogenation catalyst particles in a loop venturi reaction device comprising a hydrogenation reactor and an external circulation loop, wherein the hydrogenation reactor comprises a downward-directed gas-suspension ejector in the upper region of the hydrogenation reactor, comprising a reaction mixture inlet, a motive fluid nozzle, a suction chamber, a throat, a divergent outlet diffuser, and optionally, a convergent inlet nozzle, . The invention further relates to a loop venturi reaction device for continuously preparing hydroxylamine in a buffered aqueous phosphoric acid containing solution by hydrogenating nitrate with hydrogen at a pressure above atmospheric pressure in the presence of suspended Pd-containing hydrogenation catalyst particles and to the use of the loop venturi reaction device of the invention as stand-alone unit for the production of hydroxylamine or as a part of a chemical plant for the production of oximes, preferably as part of a chemical plant for the production of oximes, more preferably as part of a chemical plant for the production of butanone oxime, cyclopentanone oxime, cyclohexanone oxime and/or cyclodedocecanone oxime, most preferably as part of a chemical plant for the production of cyclohexanone oxime.

## Description

### FIELD OF THE INVENTION

The present invention relates to an industrial scale continuous process for preparing hydroxylamine in a buffered aqueous phosphoric acid-containing solution by hydrogenating nitrate with hydrogen in the presence of Pd-containing hydrogenation catalyst particles in a loop venturi reaction device. Furthermore, the present invention relates to a loop venturi reaction device for preparing hydroxylamine and to the use of a loop venturi reaction device as stand-alone unit for the production of hydroxylamine or as a part of a chemical plant for the production of oximes.

### BACKGROUND OF THE INVENTION

Hydroxylamine with CAS Number 7803-49-8 is a commonly used reagent in a myriad of organic and inorganic reactions. It is, in particular, suitable for use in the preparation of oximes, in particular cyclohexanone oxime, which may thereafter be converted into caprolactam via Beckmann rearrangement. Beckmann rearrangement processes for the preparation of caprolactam are generally known in the art, e.g., from Chapter "Caprolactam", in Ullmann's Encyclopedia of Industrial Chemistry (May 25, 2018), Wiley-VCH Verlag GmbH & Co. KGaA, Weinheim, Germany, electronically available via https://doi.org/10.1002/14356007.a05_031.pub3. Other oximes of which the preparation using hydroxylamine has been described include cyclodedocecanone oxime (e.g. EP1 329 448 A1) and butanone oxime.

Methods of preparing hydroxylamine are also commonly known in the art. GB 1 287 303 A, US 5,364,609 A, and US 4,328,198 A for example, relate to processes wherein nitrate or nitrogen monoxide is reduced in a phosphate buffer solution using molecular hydrogen.

The HPO^{®} and HPO^{®plus} technologies for the production of oximes (see e.g. H.J. Damme, J.T. van Goolen and A.H. de Rooij, Cyclohexanone oxime made without byproduct (NH4)2SO4, July 10, 1972, Chemical Engineering; pp 54/55 or J.T. Tinge, M.H.L. Groothaert, Y.-H. E. Sheu (2023), The Fibrant Hydranone® and HPOplus Technologies for Cyclohexanone and ε-Caprolactam Production (Case Study). An Overview of the Technology and Outlook; pp. 971-1006; Ch. 33 in: Industrial Arene Chemistry: Markets, Technologies, Sustainable Processes and Cases Studies of Aromatic Commodities (Ed. J. Mortier; electronically available via https://doi.org/10.1002/9783527827992.ch33) that are licensed by Fibrant make use of two recycling liquors - an inorganic liquid and an organic liquid - in which several reactions and operations take place. The inorganic liquid is an aqueous phosphoric acid and ammonium ions containing solution which is fed to the hydrogenation reactor, where hydroxylamine is produced. Hydroxylamine is formed via reduction of nitrate ions or nitrogen monoxide with hydrogen, which is catalyzed by a heterogeneous hydrogenation catalyst (palladium and/or platinum-containing catalyst with a solid, mostly carbon, as carrier). In general, a promoter for the heterogeneous hydrogenation catalyst is added in order to improve the performance of the catalyst. The organic liquid is an alkanone containing solution which is fed to the oximation reactor, where alkanone oxime is produced.

In the case where the hydroxylamine formation starts from a solution of phosphoric acid and nitrate the chemical reactions occurring are represented as follows:
Preparation of the hydroxylamine in the hydroxylamine formation zone:

2 H₃PO₄ + NO₃⁻ + 3 H₂ → NH₃OH⁺ + 2 H₂PO₄⁻ + 2 H₂O (1)

In acidic aqueous solutions hydroxylamine will be mainly present in the form of hydroxylammonium cations, while a very small fraction of hydroxylamine will not be protonated.

Although the HPO^{®} and HPO^{®plus} processes are known for their high selectivity of the desired product hydroxylamine, still minor amounts of by-products, i.e., ammonium, N₂O and N₂, are formed.

The by-products may be formed according to the following equations:

NO₃⁻ + 2 H⁺+ 4 H₂ → NH₄⁺ + 3 H₂O (2)

2 NO₃^{- +} 2 H⁺ + 4 H₂ → N₂O + 5 H₂O (3)

2 NO₃⁻ + 2 H⁺ + 5 H₂ → N₂ + 6 H₂O (4)

The hydroxylamine formation is heterogeneously catalyzed by Platinum or Palladium or mixtures thereof on a solid carrier and in the presence of an activator. Typically, the catalyst is present as a disperse phase of finely divided solids in a liquid reaction mixture. The resulting mixture of the first reaction is a phosphate-containing acidic aqueous solution comprising a suspension of solid catalyst particles in a hydroxylamine solution.

In the HPO^{®} and HPO^{®plus} cyclohexanone oxime processes of Fibrant, the resulting hydroxylamine solution is, after removal of the catalyst, contacted with an organic liquid containing cyclohexanone and a solvent in the oximation section. Hereby cyclohexanone reacts with hydroxylamine to form cyclohexanone oxime. Thereafter, the cyclohexanone oxime is separated from the solution of phosphoric acid and after purification converted into ε-caprolactam.

The phosphate-containing acidic aqueous solution leaving the oximation section has to be purified thoroughly to protect the catalyst in the hydroxylamine reactor. A small amount of ammonia by-product remains in solution but is prevented from building up by conversion to nitrogen in a nitrous gas (mixture of NO₂ and NO) absorber.

Water formed during the hydrogenation process and water introduced into the process via fresh nitric acid or mixtures containing nitrogen oxides are removed by evaporation.

In case nitrate is hydrogenated in the hydroxylamine formation zone, fresh nitric acid or nitrogen oxides are added, to the solution of phosphoric acid from which the cyclohexanone oxime is separated, before it is recycled to the hydroxylamine formation zone. The nitrogen oxides required in the process are produced in an ammonia combustion unit. The optionally nitric acid required in the process is produced in a nitric acid plant.

Supply of HNO₃ to make up the depletion of the source of nitrate ions:

H₃PO₄ + H₂PO₄⁻ + HNO₃ + 3 H₂ O → 2 H₃PO₄ + NO₃⁻ + 3 H₂O (5)

In the current nitrate reduction process as part of HPO^{®} and HPO^{®plus} cyclohexanone oxime processes, gaseous hydrogen is contacted in a 3-phase (i.e., gas-liquid-solid) bubble column type reactor with a circulating inorganic liquid containing nitrate ions together with a buffering acid and the catalyst. The hydrogenation of nitrate is performed under elevated pressures. The hydrogen containing gas phase is circulated over a bubble column type reactor by a circulation compressor. Fresh hydrogen is fed to the circulation gas, a small amount being withdrawn from the system to maintain a constant partial hydrogen pressure. Inert gaseous components in the fresh hydrogen and the produced gaseous by-products nitrogen (N₂) and nitrous oxide (N₂O) disappear via the gas purge. The gas-liquid suspension is circulated by the Mammoth pump principle from the gassed reactor section, over gas-liquid separators, to the filter candles in the filtration section and via a heat exchanger for the removal of heat of reaction, back to the gassed reactor section.

The preparation of hydroxylamine from nitrate ions has been known for many decades and ways to improve known preparation methods have been investigated thoroughly over the years.

WO 98/18717 A1 describes a process for producing hydroxylamine by catalytic reduction of nitrate ions and mentions the subsequent production of cyclohexanone oxime by reaction with cyclohexanone. It teaches that the selectivity of the palladium or platinum reduction catalyst can be enhanced by incorporation of a small quantity of halogen.

EP 0 773 189 A1 also describes a process for producing, hydroxylamine and subsequently cyclohexanone oxime. Also, rearrangement of cyclohexanone to ε-caprolactam is mentioned. It teaches that a nitrate reduction catalyst wherein the platinum and palladium concentrations are substantially the same, leads to improved selectivity.

EP 1 275 616 A1 also describes a process for producing hydroxylamine by catalytic reduction of nitrate ions. Efficiency of the system is improved by withdrawing gas mixture from the reaction, removing non-hydrogen compounds from this stream and recycling the hydrogen enriched phase to the reaction. US 5,155,081 A describes a platinum on graphite catalyst and its use as in a process for producing hydroxylamine, by reduction of nitrogen oxide gas, rather than from a nitrate solution. It recognizes that after time, selectivity of the catalyst deteriorates, as indicated by an increase in N₂O in the off-gas.

However, presently known industrial processes for the production of hydroxylamine, which are generally of a continuous nature, still suffer from drawbacks. Especially because there is a tendency in the ε-caprolactam production industry to increase the single line capacity to 300 and more kilotons per annum of ε-caprolactam.

Increasing the dimensions of the 3-phase bubble column type reactor, the dimensions of the heat exchanger that removes the heat of reaction and the capacity of the gas circulation compressor results in various technical issues and increased investment expenses. The construction of multiple 3-phase bubble column type reactors and auxiliary equipment that are operated in parallel is neither desirable because of increased investment and maintenance costs, including the need of additional operational attention and costs during operation of the hydrogenation section.

Hence, there exists a need to increase the production capacity of hydroxylamine using a simple apparatus design that can be scaled-up in a single line, without parallelization or numbering-up, to a production of at least 90 kiloton hydroxylamine, which is sufficient for an annual production of 300 kiloton ε-caprolactam.

In addition, there is a need to further increase the selectivity towards the conversion of nitrate into hydroxylamine and to reduce the selectivity towards the conversion into the by-products ammonium, N₂O and N₂, as this would further reduce the variable costs of the overall ε-caprolactam production process and reduce loss of valuable starting materials that are not converted into hydroxylamine, both resulting in an improved carbon footprint of the overall ε-caprolactam process.

### SUMMARY OF THE INVENTION

It is an object of the present invention, to satisfy one or more of the above-described needs and to overcome or alleviate the disadvantages associated with the prior art processes.

In particular, it is an object of the present invention to provide an improved process for the production of hydroxylamine that allows an increase in production capacity, i.e., an improved space-time yield, while satisfying one or more of the above-described needs, i.e., increased selectivity towards hydroxylamine and reduced selectivity towards the conversion into by-products and overcoming or alleviating the disadvantages associated with the prior art processes. As used herein, space-time yield is defined as the total mass of hydroxylamine produced per reactor working volume per hour.

In addition, it is a further object of the present invention to provide an improved device for the production of hydroxylamine allowing a simple apparatus design and process-scale-up in a single line, while overcoming or alleviating one or more of the disadvantages associated with the prior art devices.

One or more further objects may become apparent from the remainder of the description.

The aforementioned objects are solved by the process of claim 1 and the device of claim 9.

A continuous process for preparing hydroxylamine in a buffered aqueous phosphoric acid-containing solution by hydrogenating nitrate with hydrogen at a pressure above atmospheric pressure in the presence of suspended Pd-containing hydrogenation catalyst particles in a loop venturi reaction device comprising a hydrogenation reactor and an external circulation loop, wherein the hydrogenation reactor comprises a downward-directed gas-suspension ejector in the upper region of the hydrogenation reactor, comprising a reaction mixture inlet, a motive fluid nozzle, a suction chamber, a throat, a divergent outlet diffuser, and optionally, a convergent inlet nozzle, wherein the process comprises the steps of:
a) charging a fresh nitrate-containing buffered aqueous phosphoric acid-containing solution into the hydrogenation reactor and/or into the external circulation loop;
b) charging fresh hydrogen-containing gas into the loop venturi reaction device, preferably into the hydrogenation reactor;
c) hydrogenating nitrate charged in step a) with hydrogen charged in step b) under the influence of Pd-containing hydrogenation catalyst particles, wherein the hydrogenating is carried out at temperatures ranging from 20 to 65 °C, preferably from 25 to 55 °C, most preferably from 30 to 45 °C, to provide a reaction mixture comprising hydroxylamine-containing buffered aqueous phosphoric acid-containing solution, hydrogenation catalyst particles and hydrogen-containing gas;
d) circulating the reaction mixture through the loop venturi reaction device;
e) cooling of the reaction mixture, wherein the cooling is performed with water in an indirect heat exchanger located in the external circulation loop;
f) discharging a hydroxylamine-containing buffered aqueous phosphoric acid containing solution from the loop venturi reaction device, wherein the reaction mixture is filtered in a filtration device inserted into the external circulation loop or is passed through a line branching off from the external circulation loop connecting the external circulation loop to a filtration device prior to separate part of the hydroxylamine-containing buffered aqueous phosphoric acid-containing solution from the reaction mixture;
g) discharging a N₂O-containing gas from the loop venturi reaction device;
wherein
- the gas holdup of the reaction mixture that flows through the external circulation loop is at least 5 vol. %, preferably at least 8 vol. %; more preferably at least 12 vol. % and most preferably at least 15 vol. %,
- the phosphate concentration as determined in the hydroxylamine-containing buffered aqueous phosphoric acid containing solution discharged from the loop venturi reaction device in step f) is from 2 to 4 moles/kg, preferably from 2.8 to 3.8 moles/kg, and
- the acid concentration as determined in the hydroxylamine-containing buffered aqueous phosphoric acid containing solution discharged from the loop venturi reaction device in step f) is from 0.3 to 0.7 moles/kg, preferably from 0.4 to 0.6 moles/kg.

It was surprisingly found that carrying out a continuous process for preparing hydroxylamine in a buffered aqueous phosphoric acid-containing solution by hydrogenating nitrate with hydrogen at a pressure above atmospheric pressure in the presence of suspended Pd-containing hydrogenation catalyst particles in a loop venturi reaction device using the special processing steps and process conditions mentioned above allows to produce hydroxylamine in a straight-forward and economically reasonable manner with an improved selectivity that outperforms the selectivity of prior art processes. At the same time the selectivity towards by-products is reduced thereby reducing the costs associated with elimination of these by-products. The process of the invention allows the preparation of hydroxylamine on an industrial scale with capacities that outperform the capacities of processes of the prior art and allows to produce hydroxylamine efficiently thereby reducing the environmental burden of said product. In particular, the process of the invention allows the production of hydroxylamine that can be used for the production polyamide 6 intermediates with a carbon footprint that outperforms processes of the prior art. Thus, by applying the process of the invention not just the carbon footprint of hydroxylamine is reduced, but also of all compounds made from it, like cyclohexanone oxime, ε-caprolactam, polyamide 6 and products comprising polyamide 6 like stockings, carpets and sportwear.

The present invention also provides a loop venturi reaction device for continuously preparing hydroxylamine in a buffered aqueous phosphoric acid containing solution by hydrogenating nitrate with hydrogen at a pressure above atmospheric pressure in the presence of suspended Pd-containing hydrogenation catalyst particles, wherein the loop venturi reaction device comprises
(a) a hydrogenation reactor, preferably having a height to width ratio of at least 2, preferably of at least 4, comprising
   - an inlet for fresh hydrogen gas,
   - an outlet of N₂O-containing gas in the upper region of the hydrogenation reactor,
   - a reaction mixture outlet located in the lower region of the hydrogenation reactor,
   - a downward-directed gas-suspension ejector in the upper region of the hydrogenation reactor, wherein the downward directed gas-suspension ejector comprises a reaction mixture inlet, a motive fluid nozzle, a suction chamber, a throat, a divergent outlet diffuser, and optionally, a convergent inlet nozzle,
   - optionally, a diverter arranged below the ejector in the lower region of the hydrogenation reactor, and
   - optionally, a circulation tube arranged below the ejector in the lower region of the reactor,
   - optionally, an inlet of nitrate-containing buffered aqueous phosphoric acid-containing solution;
(b) an external circulation loop, comprising
   - a water-cooled indirect heat exchanger,
   - a circulation pump,
   - a filtration device comprising an outlet of hydroxylamine-containing buffered aqueous phosphoric acid-containing solution inserted into the external circulation loop or a line branching off from the external circulation loop connecting the external circulation loop to a filtration device comprising an outlet of hydroxylamine-containing buffered aqueous phosphoric acid containing solution; and
   - optionally an inlet of nitrate-containing buffered aqueous phosphoric acid-containing solution, and
   wherein the external circulation loop connects the reaction mixture outlet of the hydrogenation reactor with the reaction mixture inlet of gas-suspension ejector.

Surprisingly, the loop venturi reaction device of the invention can be used instead of the 3-phase bubble column type reactor in continuous industrial scale production processes, allowing a simple plant design and process-scale-up in a single line, thus without parallelization or numbering-up, to a production of at least 90 kiloton hydroxylamine and resulting in improved selectivity towards hydroxylamine, i.e., less by-products. This was particularly surprising as loop venturi reaction devices are known to be ideally suited for fast reactions involving a pure expensive gas-phase reactant and with simultaneous requirements of relatively high reaction pressure and heat removal and its most common application is in the case of a dead-end system using an expensive pure gas, while the process of the invention is a continuous process that can handle hydrogen containing gas mixtures with hydrogen contents as low as 25 percent by volume. In addition, loop venturi reaction devices have high acquisition costs and are therefore usually applied in multipurpose, campaign-based manufacture of high-value chemicals, while hydroxylamine produced by the process of the invention is just a cheap intermediate for the bulk chemical ε-caprolactam, the monomer of polyamide 6, also known as nylon 6. However, due to the improved selectivity towards hydroxylamine and the possibility of simple process scale-up in a single line the replacement of the 3-phase bubble column reactor in existing production facilities is still profitable and results in an overall economic benefit.

Advantageous embodiments of the invention are indicated in the dependent claims and are explained in more detail in the following.

### DETAILED DESCRIPTION OF THE INVENTION

The invention provides a continuous process for preparing hydroxylamine in a buffered aqueous phosphoric acid-containing solution by hydrogenating nitrate with hydrogen at a pressure above atmospheric pressure in the presence of suspended Pd-containing hydrogenation catalyst particles in a loop venturi reaction device.

The process of the invention is a continuous process. The term "continuous process" as used herein, refers to a process, in which the feed flows, i.e., a hydrogen-containing gas flow from outside the loop venturi reaction device, and a nitrate-containing buffered aqueous phosphoric acid containing solution from outside the loop venturi reaction device, are charged without interruption in the loop venturi reaction device and the product flow, i.e., a hydroxylamine-containing buffered aqueous phosphoric acid-containing solution to outside the loop venturi reaction device is discharged without interruption to outside the loop venturi reaction device. "In a continuous mode" can thus be understood to be the opposite of a "batch mode". A hydroxylamine-containing buffered aqueous phosphoric acid-containing solution as described herein is an aqueous solution comprising hydroxylamine mainly in the form of hydroxylammonium cations, nitrate ions, and buffered aqueous phosphoric acid. A buffered aqueous phosphoric acid-containing solution as used herein is a solution comprising aqueous hydrogen phosphates and ammonium ions.

The process of the invention is carried out in a loop venturi reaction device. A loop venturi reaction device comprises a circulating reaction mixture, to which a nitrate-containing buffered aqueous phosphoric acid-containing solution and a hydrogen-containing gas flow are fed and from which a hydroxylamine-containing buffered aqueous phosphoric acid-containing solution as product flow and a gas flow containing by-product N₂O are withdrawn. The device usually comprises a gas-suspension ejector to generate a 3-phase system that comprises a buffered aqueous phosphoric acid-containing solution, a hydrogen-containing gas phase and solid Pd-containing hydrogenation catalyst particles. The loop venturi reaction device used in the continuous process for the hydrogenation of nitrate according to the present invention comprises a hydrogenation reactor, an external circulation loop, an indirect heat exchanger and a filtration device, wherein the filtration device is inserted into the external circulation loop or is passed through a line branching off from the external circulation loop connecting the external circulation loop to a filtration device, wherein the hydrogenation reactor comprises a downward-directed gas-suspension ejector in the upper region of the hydrogenation reactor comprising a reaction mixture inlet, a motive fluid nozzle, a suction chamber, a throat, a divergent outlet diffuser and optionally, a convergent inlet nozzle,. The loop venturi reaction device may comprise further parts that are usual in the art and known to the skilled person. Preferably, the loop venturi reaction device is a loop venturi reaction device according to the invention, which is further described hereinbelow. It is to be understood that all features described in the context of the loop venturi reaction device of the invention below are equally applicable to the venturi device used in the process of the invention.

In step a) of the process of the invention, a fresh nitrate-containing buffered aqueous phosphoric acid containing solution is charged into the hydrogenation reactor and/or into the external circulation loop of the loop venturi reaction device.

In step b) of the process of the invention fresh hydrogen-containing gas is charged into the loop venturi reaction device, preferably into the hydrogenation reactor. Fresh hydrogen-containing gas can be fed into the loop venturi reaction device in a manner and at a concentration (partial hydrogen pressure) known per se.

In general, the hydrogen-containing gas that is fed into the loop venturi reaction device is not entirely pure, but can contain one or more inerts, like nitrogen, water, helium, argon, carbon dioxide, methane, ethane and propane. The types and the concentrations of these inerts are very much depending on the technology used to prepare and purify the hydrogen-containing gas. Hydrogen that is not entirely pure is preferred for economic reasons. In general, the hydrogen concentration of hydrogen-containing gas can be more than 50 vol.%, preferably more than 85 vol.%, more preferably more than 95 vol.% and most preferably more than 99 vol.%. High partial hydrogen pressures in the loop venturi reaction device are preferred, because it has a positive impact on both the activity and the selectivity of the Pd-containing hydrogenation catalyst particles.

The hydrogen partial pressure in the hydrogenation reactor during the preparation of hydroxylamine, can be adjusted by manipulating the ingoing flow of fresh hydrogen-containing gas in step b) and the gas flow of N₂O-containing gas that is discharged in step g). The hydrogen partial pressure of the N₂O-containing gas that is discharged is lower than the hydrogen partial pressure of ingoing flow of fresh hydrogen-containing gas, due to consumption of hydrogen and the formation of by-products. By increasing the incoming flow of fresh hydrogen-containing gas in step b) and the gas flow of N₂O-containing gas that is discharged in step g), the partial pressure of hydrogen in the hydrogenation reactor will increase. While reducing the incoming fresh hydrogen-containing gas flow in step b) and the venting N₂O-containing gas flow in step g) will reduce the hydrogen partial pressure in the hydrogenation reactor.

The total gas pressure in the hydrogenation reactor can be varied within wide limits. A high total gas pressure is advantageous because it limits the gas flow of N₂O-containing gas that is discharged from the loop venturi reaction device, thereby increasing the utility rate of hydrogen, i.e., less hydrogen is lost in the purge stream leaving the loop venturi reaction device. On the other hand, very high pressures in the hydrogenation reactor are not desired due to high equipment costs. A low total gas pressure in the hydrogenation reactor is advantageous because of equipment costs. On the other hand, it requires a large gas flow of N₂O-containing gas that is discharged from the loop venturi reaction device, thereby reducing the utility rate of hydrogen, i.e., more hydrogen is lost in the purge stream leaving the loop venturi reaction device. According to a preferred embodiment of the invention the total gas pressure in the hydrogenation reactor during the reaction is ranging from 1 to 200 bar, preferably from 2 to 100 bar, more preferably from 3 to 75 bar, and most preferably from 10 to 50 bar.

Very good results regarding selectivity towards hydroxylamine and productivity of the catalyst were obtained by maintaining the hydrogen partial pressure in the hydrogenation reactor during the preparation of hydroxylamine within the range of from 1 bar to 40 bar, in particular in the range of from 5 bar to 25 bar, most particular from 10 bar to 20 bar.

The inert gas partial pressure in the hydrogenation reactor can be varied within wide limits. A high inert gas partial pressure is advantageous because it limits the gas flow of N₂O-containing gas that is discharged from the loop venturi reaction device, thereby increasing the utility rate of hydrogen, i.e., less hydrogen is lost in the purge stream leaving the loop venturi reaction device. On the other hand, high inert gas partial pressures in the hydrogenation reactor are not desired due to high equipment costs. A low inert gas partial pressure in the hydrogenation reactor is advantageous because equipment costs. On the other hand, it requires a large gas flow of N₂O-containing gas that is discharged from the loop venturi reaction device, thereby reducing the utility rate of hydrogen, i.e., more hydrogen is lost in the N₂O-containing gas leaving the loop venturi reaction device. Especially good results were obtained by ranging the inert gas partial pressure in the hydrogenation reactor during the reaction from 0.5 to 50 bar, preferably from 2 to 40 bar, most preferably from 5 to 30 bar.

In step c) of the process of the invention nitrate charged in step a) is hydrogenated with hydrogen charged in step b) under the influence of Pd-containing hydrogenation catalyst particles, wherein the hydrogenating is carried out at temperatures ranging from 20 to 65 °C, preferably from 25 to 55 °C, most preferably from 30 to 45 °C, to provide a reaction mixture comprising hydroxylamine-containing buffered aqueous phosphoric acid-containing solution, hydrogenation catalyst particles and hydrogen-containing gas.The hydrogenation reaction results in the generation of heat of reaction that is at least partially stored in the buffered aqueous phosphoric acid-containing solution (i.e., the solution is warmed). Apart from hydroxylamine nitrous oxide (N₂O) is formed during the reaction.

The Pd-containing hydrogenation catalyst particles applied in the preparation of hydroxylamine according to the process of the invention mostly consists of Pd as active component on a carrier material such as for instance carbon. The catalyst may be activated by the presence of one or more catalyst activators. The catalyst activator may be an element from the group comprising Cu, Ag, Cd, Hg, Ga, In, Ti, Ge, Sn, Pb, As, Sb and Bi. Most preferably the catalyst activator is Ge. Compounds containing the elements in question may also be used as catalyst activators, for example oxides, nitrates, phosphates, sulphates, halogenides, and acetates. The elements or their compounds can be part of the Pd-containing hydrogenation catalyst particles as described in US 3,767,758 A or they can be added to the reaction medium. Preferably, the Pd-containing hydrogenation catalyst comprises Pd or a combination of Pd and Pt on a support. The Pd:Pt weight ratio may vary, although in general the preference is for pure Pd. The pure Pd may contain some Pt impurities. Preferably the Pd-containing hydrogenation catalyst particles comprise less than 25 wt% Pt, more preferably less than 5 wt% Pt, even preferably less than 2 wt% Pt. Preferably, the support comprises carbon (e.g., graphite, carbon black, or activated carbon) or alumina support, more preferably graphite or activated carbon. The Pd-containing hydrogenation catalyst particles preferably comprise between 1 to 25 wt% Pd or Pd and Pt, more preferably between 5 to 15 wt% Pd or Pd and Pt, relative to the total weight of support plus catalyst particles. Generally, the Pd-containing hydrogenation catalyst can be present in the reaction mixture in an amount of 0.05 to 25 wt%, preferably in an amount of 0.2 to 15 wt%, more preferably in an amount of 0.5 to 5 wt% relative to the total reaction mixture. Preferably, the Pd-containing hydrogenation catalyst particles have in general an average size of between 1 and 150 µm, more preferably between 5 and 100 µm, even more preferred between 5 and 60 µm and most preferably between 5 and 40 µm as determined by laser diffraction. By "average particle size" is meant that 50 vol% of the particles are larger than the specified diameter.

Preferably, the catalyst activator is present in an amount of between 0.01 and 100 mg/g Pd-containing hydrogenation catalyst particles, preferably between 0.05 and 50 mg/g Pd-containing hydrogenation catalyst particles, more preferably between 0.1 and 10 mg/g Pd-containing hydrogenation catalyst particles, most preferably between 1 and 7 mg/g Pd-containing hydrogenation catalyst particles.

In accordance with the invention, promoter or Pd-containing hydrogenation catalyst particles can be continuously or intermittently added to the loop venturi reaction device, preferably the promoter and the Pd-containing hydrogenation catalyst particles are intermittently added to the loop venturi reaction device. Adding promoter is in particular advantageous for increasing the hydroxylamine production rate in terms of product rate per kg catalyst in the loop venturi reaction device, as it increases the activity of the catalyst already present in the loop venturi reaction device. Adding Pd-containing hydrogenation catalyst particles is in particular useful for increasing the hydroxylamine production rate without impairing the selectivity. If desired, promoter and Pd-containing hydrogenation catalyst particles can be added simultaneously. Preferably, Pd-containing hydrogenation catalyst particles are added together with promoter, whereas promoter may also advantageously be added without Pd-containing hydrogenation catalyst particles. The location of the dosing point of the Pd-containing hydrogenation catalyst particles and/or promoter can be anywhere in the loop venturi reaction device. The skilled person is able to select the optimal location for the dosing point of the Pd-containing hydrogenation catalyst particles and/or promoter in the loop venturi reaction device. Preferably, the Pd-containing hydrogenation catalyst particles and/or the promotor are introduced into, i.e., dosed into, the hydrogenation reactor. As an equally preferred alternative, the Pd-containing hydrogenation catalyst particles and/or promotor can be introduced into, i.e., dosed into, the external circulation loop.

The addition of Pd-containing hydrogenation catalyst particles and/or promotor can be done in a continuous mode or in a batch mode. The activity and selectivity of the catalyst-promotor system remain at a high level for a long time, meaning that the addition of extra amounts of Pd-containing hydrogenation catalyst particles and/or promotor is not necessary or only necessary to a very limited extent. According to a preferred embodiment of the invention the charging of Pd-containing hydrogenation catalyst particles into the hydrogenation reactor [A] and/or the external circulation loop is performed in a batch-wise mode.

In step d) of the process of the invention the reaction mixture is circulated through the loop venturi reaction device. The reaction mixture is circulated through the loop by means of a circulation pump. Preferably, the circulation rate of the reaction mixture ranges from 10 and 500 times per hour, preferably from 15 to 300 times per hour. Such a circulation rate has proven to be particularly advantageous for the hydrogen gas-liquid mass transfer as it allows vigorous mixing of the gas and liquid phases. The reaction mixture that is circulated through the external circulation loop of the loop venturi reaction device is a 3-phase liquid-gas-solid system, comprising a hydroxylamine-containing buffered aqueous phosphoric acid-containing solution, a hydrogen-containing gas phase and solid Pd-containing hydrogenation catalyst particles. Very good results in improving or at least maintaining the catalyst performance in terms of activity and selectivity for a longer period of time were obtained for reaction mixtures comprising a minimum amount of gas phase. In addition, the circulation of a 3-phase system avoids or reduces the need to separate gas bubbles from the suspension of buffered aqueous phosphoric acid-containing solution and solid particles of the hydrogenation catalyst before entering the external circulation loop. Therefore, according to the invention the gas holdup of the reaction mixture that flows through the external circulation loop is at least 5 vol. %, preferably at least 8 vol. %, more preferably at least 12 vol % and most preferably at least 15 vol. %.

In step e) of the process of the invention the reaction mixture is cooled, wherein the cooling is performed with water in an indirect heat exchanger located in the external circulation loop. The cooling removes the heat of reaction that was stored in the buffered aqueous phosphoric acid-containing solution. In general, the heat transfer in the indirect heat exchanger is improved by increasing the velocity of the reaction mixture. Preferably, the velocity of the reaction mixture inside the tubes of a shell and tube indirect heat exchanger is at least 1 m/s.

According to step f) of the process of the invention a hydroxylamine-containing buffered aqueous phosphoric acid-containing solution is discharged from the loop venturi reaction device, wherein the reaction mixture is filtered in a filtration device inserted into the external circulation loop or is passed through a line branching off from the external circulation loop connecting the external circulation loop to a filtration device prior to separate part of the hydroxylamine-containing buffered aqueous phosphoric acid-containing solution from the reaction mixture. In accordance with the invention the hydroxylamine concentration as determined in the hydroxylamine-containing buffered aqueous phosphoric acid-containing solution that is discharged from the loop venturi reaction device in step f) can be chosen within a wide range. The hydroxylamine concentration in the hydroxylamine-containing buffered aqueous phosphoric acid-containing solution that is discharged from the loop venturi reaction device in step f) can, e.g., be varied in the range of 0.1 to 2.5 mol/kg. Extremely good results were obtained at values in the range of 0.50 to 2 mol/kg, in particular at concentrations of about 1 mol/kg of buffered aqueous phosphate containing solution.

According to step g) of the process of the invention a N₂O-containing gas is discharged from the loop venturi reaction device. Continuously discharging a N₂O-containing gas from the loop venturi device is advantageous because it prevents the continuous build-up of gaseous non-hydrogen compounds in the loop venturi reaction device, thereby reducing the hydrogen partial pressure, in the event that the loop venturi reaction device is operated at a fixed total pressure. The build-up of gaseous non-hydrogen compounds is due to the following two processes. Firstly, hydrogen is consumed in the nitrate hydrogenation, while inerts present in the feed are not consumed. The ratio hydrogen to non-hydrogen compounds therefore decreases over time. Secondly, inerts like nitrogen and nitrous oxide, N₂O are formed as by-products during the hydrogenation of nitrates. And again, the ratio hydrogen to non-hydrogen compounds therefor decreases over time. It is well-known that hydrogen and N₂O can form explosive mixtures. Because of this nitrate hydrogenations are always operated well below the Lower Explosive Limit (LEL) of this gas mixture. This is another reason that a loop venturi reaction device has an outlet through which gaseous non-hydrogen compounds including N₂O are discharged. In a preferred embodiment of the process according to the invention, the nitrous oxide concentration in the N₂O-containing gas that is discharged in step g) is ranging from 0 to 2 vol. %, in particular from 0.01 to 1 vol. %, more in particular from 0.05 to 0.5 vol. %, most particularly from 0.08 to 0.3 vol. %. The economics and the sustainability of the process can be improved by recovering hydrogen gas from the N₂O-containing gas discharged in step g) and at least partly re-using the recovered hydrogen gas, preferably at least partly re-using the recovered hydrogen gas for preparing hydroxylamine in a process according to the invention. It is desired to remove at least part of the N₂O from the N₂O-containing gas discharged in step g) before it is re-used for preparing hydroxylamine in a process according to the invention. According to a preferred embodiment of the invention the N₂O-containing gas that is discharged in step g) is purified and at least part of the discharged N₂O-containing gas is re-used for the hydrogenation reaction, preferably wherein the purification is performed by adsorption or membrane separation, whereby at least part of the N₂O is removed from the N₂O-containing gas.

The hydrogenation in the process according to the invention is carried out in a buffered aqueous phosphoric acid-containing solution. This solution provides phosphate as phosphoric acid or as hydrogen phosphate salt, which may be formed by adjusting the pH of a buffered phosphoric acid-containing solution with an appropriate base, such as a hydroxide or ammonia. According to the invention the phosphate concentration as determined in the hydroxylamine-containing buffered aqueous phosphoric acid containing solution discharged from the loop venturi reaction device in step f) is ranging from 2 to 4 moles/kg, preferably from 2.8 to 3.8 moles/kg. This has been proven to be particularly advantageous as phosphate concentrations below 2 moles/kg will result in more diluted hydroxylamine contents and phosphate concentrations above 4 moles/kg will rise the crystallization temperature to undesired levels.

In accordance with the invention the acid concentration (sometimes called free acid concentration) as determined in the hydroxylamine-containing buffered aqueous phosphoric acid containing solution discharged from the loop venturi reaction device in step f) is in the range of from 0.3 to 0.7 mol/kg, more in particular in the range of from 0.4 to 0.6 mol/kg.

Acid concentration is the molar concentration of H⁺ in mol/kg in the liquid leaving the loop venturi reaction device and phosphate concentration is the total concentration of phosphate (including phosphate in H₃PO₄, monohydrogen phosphate and dihydrogen phosphate) in mol/kg in the liquid leaving the loop venturi reaction device.

Acid, hydroxylamine and phosphate concentrations are all determined by equilibrium titration of one sample, by subsequently titrating a sample of the buffered aqueous phosphoric acid-containing solution at 25 °C with 0.25 N aqueous NaOH solution) to get the acid at the first equilibrium point (at a pH of about 4.2); next molar excess of acetone is added to the sample, to convert hydroxylamine into an oxime and H⁺, and equilibrium titration is continued so as to subsequently reach three further equivalence points, the first of which is corresponding to the free acid coming from hydroxylamine (and thus provides the value for hydroxylamine in the sample); the second of which provides the value for phosphate concentration, and the last of which provides a value for ammonium. The latter value, however, is not needed here.

In accordance with the invention the nitrate concentration as determined in the hydroxylamine-containing buffered aqueous phosphoric acid containing solution leaving the loop venturi reaction device in step f) can be varied within a wide range.

Usually, the nitrate concentration as determined in the hydroxylamine-containing buffered aqueous phosphoric acid containing solution leaving the loop venturi reaction device in step f) is 2 mol/kg or less, in particular 1.0 mol/kg or less. In a preferred embodiment, the nitrate concentration as determined in the hydroxylamine-containing buffered aqueous phosphoric acid containing solution leaving the loop venturi reaction device in step f) is 0.9 mol/kg or less, in particular 0.8 mol/kg or less. Particularly good results have been achieved with a nitrate concentration as determined in the hydroxylamine-containing buffered aqueous phosphoric acid containing solution leaving the loop venturi reaction device in step f) of about 0.70 or less. Usually, the nitrate concentration as determined in the hydroxylamine-containing buffered aqueous phosphoric acid containing solution leaving the loop venturi reaction device in step f) is at least 0.3 mol/kg, in particular at least 0.4 mol/kg. Preferably, the nitrate concentration as determined in the hydroxylamine-containing buffered aqueous phosphoric acid containing solution leaving the loop venturi reaction device in step f) is at least 0.45 mol/kg, more preferably at least 0.50 mol/kg.

In accordance with the invention the molar ratio of nitrate to phosphate in the hydroxylamine-containing buffered aqueous phosphoric acid containing solution that is discharged from the loop venturi reaction device in step f) is usually at least 0.05 mol/kg, preferably at least 0.10 mol/kg. Excellent results are achieved at values of at least 0.15 mol/kg, more in particular of at least 0.20 mol/kg. The molar ratio of nitrate to phosphate preferably is 0.40 mol/kg or less, in particular 0.35 mol/kg or less, more in particular 0.30 mol/kg or less.

In accordance with the invention the ratio of the nitrate in the hydroxylamine-containing buffered aqueous phosphoric acid containing solution that is discharged from the loop venturi reaction device in step f) and the nitrate concentration in the fresh nitrate-containing buffered aqueous phosphoric acid-containing solution charged into the loop venturi reaction device in step a) can be chosen within a wide range. Experiments have shown that depletion of nitrate in the reaction mixture has a negative impact on productivity of the catalyst. Experiments have shown that very good results can be obtained in case the ratio of the nitrate concentration expressed in mol/kg solution in the hydroxylamine-containing buffered aqueous phosphoric acid-containing solution discharged from the loop venturi reaction device in step f) and the nitrate concentration expressed in mol/kg solution in the nitrate-containing buffered aqueous phosphoric acid-containing solution charged into the loop venturi reaction device in step a) ranges from 1:2 to 1:6, more preferably from 1:2.5 to 1:5.

Another object of the invention is a loop venturi reaction device for continuously preparing hydroxylamine in a buffered aqueous phosphoric acid-containing solution by hydrogenating nitrate with hydrogen at a pressure above atmospheric pressure in the presence of suspended Pd-containing hydrogenation catalyst particles. All device features specifically described in connection with the device below also correspond to specific embodiments of the process of the invention and vice versa. Thus, the device is preferably suitable for carrying out the process of the invention and it is to be understood that what has been described in connection with the process of the invention equally applies to the device embodiments.

The loop venturi reaction device of the invention comprises a hydrogenation reactor, preferably having a height to width ratio of at least 2, preferably of at least 4, comprising an inlet for fresh hydrogen gas, an outlet of N₂O-containing gas in the upper region of the hydrogenation reactor, a reaction mixture outlet located in the lower region of the hydrogenation reactor, a downward-directed gas-suspension ejector in the upper region of the hydrogenation reactor, wherein the downward directed gas-suspension ejector comprises a reaction mixture inlet, a motive fluid nozzle, a suction chamber, a throat, a divergent outlet diffuser, and optionally, a convergent inlet nozzle, optionally, a diverter arranged below the ejector in the lower region of the hydrogenation reactor, and optionally, a circulation tube arranged below the ejector in the lower region of the reactor, optionally, an inlet of nitrate containing buffered aqueous phosphoric acid containing solution.

The hydrogenation reactor as part of a loop venturi reaction device comprises a reaction vessel and a downward-directed gas-suspension ejector in the upper region of the hydrogenation reactor. The reaction vessel can be any type of reaction vessel suitable for containing a suspension of a buffered aqueous phosphoric acid-containing solution and Pd-containing hydrogenation catalyst particles and a hydrogen-containing gas. Preferably, the reaction vessel is a vertically upright column whose length is greater than its diameter, more preferably length to diameter ratio of the column is at least 2, most preferably length to diameter ratio of the column at least 4. This is advantageous, because the hydrogenation reactor needs to be operated under pressurized conditions, i.e. at pressures above atmospheric pressure. The terms "length" and "diameter" as used herein are the internal length of the column and the internal diameter of the column, respectively. Preferably, the reaction vessel does not contain any built-in devices, like baffles, circulation tube(s), diverter(s), coil(s) or heat exchanger(s) coils, thereby minimizing the required volume of the reaction vessel that needs to be operated under at pressures above atmospheric pressure. It is to be understood that the dimensions of the reaction vessel correspond to the dimensions of the hydrogenation reactor, therefore the terms reaction vessel and hydrogenation reactor are used interchangeable herein.

During operation of the hydrogenation reactor the lower part of the reaction vessel usually contains as reaction mixture a 3-phase system, i.e., a suspension of a hydroxylamine-containing buffered aqueous phosphoric acid- containing solution and Pd-containing hydrogenation catalyst particles constituting a continuous phase, and hydrogen-containing gas bubbles, while the upper part of the reaction vessel is mainly containing a hydrogen-containing gas. The upper part of the reaction mixture might be foamy and thus have a very high volumetric gas fraction.

The hydrogenation reactor comprises a downward-directed gas-suspension ejector in the upper region of the hydrogenation reactor, wherein the downward directed gas-suspension ejector comprises a reaction mixture inlet, a motive fluid nozzle, a suction chamber, a throat, a divergent outlet diffuser and optionally, a convergent inlet nozzle. The primary purpose of the gas-suspension ejector is creating dispersion of hydrogen-containing gas in the suspension of a buffered aqueous phosphoric acid-containing solution and Pd-containing hydrogenation catalyst particles. Gas-suspension ejectors have the advantage of rapid gas-liquid mass transfer. By incorporating a jet, high-energy dissipation rates can easily be achieved. These properties allow that higher productivities can be obtained by using a loop venturi reaction device of the invention under similar conditions, such as catalyst loading, partial hydrogen pressure and temperature, compared to the prior art devices such as stirred tanks and bubble columns. Alternatively, the higher productivities in the loop venturi reaction device according to the invention can also be exchanged for lower catalyst loadings while maintaining productivity. Finally, higher selectivity towards hydroxylamine can be obtained in the loop venturi reaction device of the invention for the hydrogenation of nitrate compared to prior art devices such as stirred tanks and bubble columns.

Without wishing to be bound by theory the inventors believe that this is a result of the improved heat transfer in the heat exchangers inserted in the external circulation loop of a loop venturi reaction device of the present invention compared to the heat transfer in prior art devices such as stirred tank reactors and bubble column reactors. The improved heat transfer allows that the nitrate hydrogenation can be carried out at lower reaction temperatures for a fixed amount of cooling water, thereby enhancing the selectivity towards hydroxylamine. Gas-liquid mass transfer of hydrogen in the loop venturi reaction device of the invention is improved compared to the gas-liquid mass transfer in prior art devices such as stirred tanks reactors and bubble column reactors. This ensures that higher hydrogen concentrations in the reaction mixture are obtained under otherwise similar conditions, such as catalyst loading, partial hydrogen pressure and temperature, compared to prior art devices such as stirred tank reactors and bubble column reactors, thereby enhancing the selectivity towards hydroxylamine.

Preferably, the gas-suspension ejector is a self-priming gas-suspension ejector. The gas-suspension ejector comprises a reaction mixture inlet, a motive fluid nozzle also called liquid nozzle, a suction chamber, optionally, a convergent inlet nozzle, a throat and a divergent outlet diffuser. This type of gas-suspension ejector is often called venturi ejector. The circulation pump inserted in the external circulation loop supplies fluid through the motive fluid nozzle thereby obtaining a jet with a high velocity. This creates a reduced pressure and consequently hydrogen containing gas is sucked into the suction chamber and taken with the jet into the convergent inlet nozzle and into the throat, where an intensive mixing of the phases occurs. Then the 3-phase reaction mixture containing very fine gas bubbles flows through the divergent outlet diffuser and into the main body the hydrogenation reactor. Preferably, the hydrogen containing gas that is sucked into the suction chamber is a mixture of fresh hydrogen-containing gas from outside the loop venturi reaction device and hydrogen-containing gas drawn from the upper part of the hydrogenation reactor. It has been proven that the outflow rate of the three-phase flow, which comprises a suspension of a hydroxylamine-containing buffered aqueous phosphoric acid-containing solution, a hydrogen-containing gas phase and solid particles of the hydrogenation catalyst, leaving the liquid nozzle, determines the rate of aspiration of gas in the suction chamber. It was shown that at exit velocities from the liquid nozzle of less than 10 m/s, the suction of gas into the suction chamber was insufficient, causing the observed rate of nitrate hydrogenation to be very limited and the selectivity to be reduced to the desired hydroxylamine compound was too low, causing many by-products to be formed. For exit velocities from the liquid nozzle higher than 10 m/s, a positive correlation between the exit velocity and the observed rate of nitrate hydrogenation was observed, while the selectivity for hydroxylamine formation reached a desired level. For liquid nozzle exit velocities higher than 500 m/s, no improvements were observed in either nitrate hydrogenation rate or selectivity toward hydroxylamine. In a preferred embodiment of the invention, the exit velocity of the reaction mixture leaving the liquid nozzle is at least 10 m/s, preferably more than 20 m/s, and the hydrogen-containing gas is sucked from the suction chamber.

Preferably, the gas-suspension ejector having a downward-facing jet nozzle is arranged in the upper part of the hydrogenation reactor through which the suspension of a buffered aqueous phosphoric acid-containing solution and Pd-containing hydrogenation catalyst particles and hydrogen-containing gas are fed in the hydrogenation reactor. The outlet of the divergent outlet diffuser can be either above or below the surface of the 3-phase system, i.e., a suspension of a buffered aqueous phosphoric acid containing solution and hydrogenation catalyst particles, and hydrogen containing gas bubbles, in the main body of the hydrogenation reactor.

The design of the gas-suspension ejector is important as for creating primary dispersion in the nozzle while the kinetic energy of the liquid jet is important for the entrainment of gas and subsequent dispersion. The skilled person is able to select or design the optimal type of a gas-suspension ejector.

Optionally, the hydrogenation reactor is equipped with more than one gas-suspension ejector. Preferably, these gas-suspension ejectors can be operated in parallel. Alternatively, instead of applying a gas-suspension ejector containing a single-channel, with one nozzle and one diffuser, a multi-channel ejector with multiple, separate channels housed inside a single, common ejector body a can be applied. Advantageous of multiple gas-suspension ejectors or channels are increased capacities and reduced sizes, i.e., shorter.

Optionally, a diverter (also known as flow reversal or diversion pan) is arranged below the ejector in the lower region of the reactor for diverting the reaction mixture in reaction vessel thereby improving the mixing efficiency of the reaction mixture in the reaction vessel. The liquid circulation mode and high degree of turbulence in the reaction vessel provide favorable conditions for catalyst suspension, thus avoiding settling of catalyst particles. Another advantage of a diverter is that the gas hold-up in the reaction mixture leaving the reaction vessel to the external circulation loop is reduced.

Optionally, in addition to a diverter also a circulation tube is arranged below the ejector in the lower region of the reactor for diverting the reaction mixture in reaction vessel thereby further improving the mixing efficiency of the reaction mixture in the reaction vessel.

The external circulation loop comprised in the loop venturi reaction device of the invention comprises a water-cooled indirect heat exchanger, a circulation pump, a filtration device comprising an outlet of hydroxylamine-containing buffered aqueous phosphoric acid-containing solution inserted into the external circulation loop or a line branching off from the external circulation loop connecting the external circulation loop to a filtration device comprising an outlet of hydroxylamine-containing buffered aqueous phosphoric acid containing solution; and optionally an inlet of nitrate-containing buffered aqueous phosphoric acid-containing solution. The external circulation loop connects the reaction mixture outlet of the hydrogenation reactor with the reaction mixture inlet of the gas-suspension ejector.

The term "heat exchanger" as used herein is a device for transferring heat from one fluid stream to another. A heat exchanger may be direct (wherein the fluid streams are mixed) or indirect (wherein the fluid streams remain separated by a dividing wall). All heat exchangers referred to herein are indirect heat exchangers. An indirect heat exchanger comprises at least two chambers with a dividing wall. In its simplest form it comprises two chambers. Heat is transferred from a fluid in a first chamber, through the dividing wall, to a fluid in a second chamber. Each chamber independently may have a long pathway, and a large surface area to volume ratio to facilitate heat transfer. Indirect heat exchangers are well-known to the person of skill in the art. The purpose of the heat exchanger is to transfer sensible heat of the hydroxylamine-containing buffered aqueous phosphoric acid-containing solution to a cooling fluid, thereby lowering the temperature of the hydroxylamine-containing buffered aqueous phosphoric acid containing solution. This can be done by one or more heat exchangers that are operated in series and/or in parallel. According to a preferred embodiment of the loop venturi reaction device of the invention, the water-cooled indirect heat exchanger is selected from a shell and tube type heat exchanger or a plate type heat exchanger, preferably a shell and tube type heat exchanger. A plate type indirect heat exchanger is preferred, because it is capable of handling a large flow and allows a small Log Mean Temperature Difference (LMTD) between the liquids between which heat is transferred. A shell and tube indirect heat exchanger is particularly preferred, because it is robust due to its shape. Even more preferred is a multi-tubular shell and tube indirect heat exchanger, wherein the heat exchanger tubes are arranged in a bundle of tubes.

According to the invention, particularly good results can be achieved when the heat exchanger is inserted in the external circulation loop. This has the advantage that the heat exchanger can be built as large as needed and is not limited by the working volume of the reactor. In addition, due to higher flow rates the heat transfer on process side of a heat exchanger inserted in the external circulation loop can be much higher than the heat transfer of a heat exchanger inserted in the reactor, meaning that less heat-transferring surface area is required, and the heat exchanger therefore can be made smaller. Furthermore, a heat exchanger inserted in the external circulation loop is easier accessible for maintenance and repair. And the full heat exchanger surface area on process side is always available for heat transfer independent of the working volume of the reactor. And finally, the costs of a heat exchanger inserted in the external circulation loop can be lower than a heat exchanger built into the reactor.

In principle, any type of coolant can be used to remove the heat in indirect heat exchangers of the loop venturi reaction device. However, because of costs cooling water is used as coolant in the indirect heat exchangers of the loop venturi reaction device of the invention. Once-through cooling is the simplest and earliest form of water cooling. In this case, water is extracted from a river, a lake, groundwater or seawater and is pumped to the heat exchanger. The heated water is returned to the source of water from which it was extracted. Alternatively, a cooling circuit is in place to recirculate the cooling water. In general, cooling water is not plain water but contains often conditioners.

The main purpose of the circulation pump in the external circulation loop of the loop venturi reactor of the invention, also known as a circulator pump, is to create a large flow rate of reaction mixture through the external circulation loop as it creates the power input of the ejector. The circulating fluid is forced through an ejector nozzle where the fluid is accelerated into a jet, which due to its momentum entrains reaction gas into the mixing tube. In addition, large flow rates of reaction mixture through the external circulation loop and through the heat exchanger(s) improve the heat transfer (process side) in the indirect heat exchangers. Furthermore, the settling of Pd-containing hydrogenation catalyst particles in the external circulation loop is reduced at large flow rates of reaction mixture due to high degrees of turbulence, which means less fouling will occur. Finally, the operation of a crossflow filtration unit, which is preferably used as a filtration device inserted into the external circulation loop, is improved by increasing flow rates due to reduced build-up of any filter cake on the surface of the filter, which hinders the filtrate from passing through the filter medium.

The circulation pump in the external circulation loop can be any type of pump suitable for pumping the reaction mixture comprising hydroxylamine-containing buffered aqueous phosphoric acid-containing solution, Pd-containing hydrogenation catalyst particles and hydrogen-containing gas. Preferably the hydrogen containing gas is present in the form of gas bubbles. Preferably the circulation pump is specialized in providing a large flow rate and a low head. The skilled person is able to select the optimal type of circulation pump, since selection of pumps is a standard activity. A unique impeller and a special hydrodynamic pump house profile allow pumping of liquid with a high solid content and high gas loads, without the aid of an inducer and thus avoiding abrasion problems where heterogeneous catalysts are used. Preferably, a centrifugal pump without the aid of an inducer and thus avoiding abrasion problems where heterogeneous catalysts is selected.

The loop venturi reaction device of the invention comprises a filtration device comprising an outlet of hydroxylamine-containing buffered aqueous phosphoric acid-containing solution inserted into the external circulation loop or a line branching off from the external circulation loop connecting the external circulation loop to a filtration device comprising an outlet of hydroxylamine-containing buffered aqueous phosphoric acid-containing solution. The filtration device serves to remove the Pd-containing hydrogenation catalyst particles from the hydroxylamine- containing buffered aqueous phosphoric acid-containing solution before the solution is contacted with an organic liquid containing cyclohexanone and a solvent in the oximation section. This is advantageous because the recovered Pd-containing hydrogenation catalyst particles can be reused in the hydrogenation process, and it prevents down-stream blocking and other types of process disturbances. The filtration device can be a cross-flow filtration device or a dead-end filtration device.

In a dead-end filtration device, the feed is passed through a membrane or bed, the Pd-containing hydrogenation particles being deposited on the filter material and the filtrate being released at the other end. In general, effective functioning of such a filtration device even requires the formation of a filter cake. Often, the effective pore size of a filter cake is smaller than that of the filter alone, improving particle-fluid separation filter cake build-up. However, the filter cake must not be allowed to become too thick as otherwise the filtrate flux is restricted too much and because in the filter cake in the absence of hydrogen decomposition of the hydroxylamine can occur. The filter cake will therefore have to be removed regularly. Removal of filter cake, for instance by backwashing, is laborious and is disturbing in a continuously operated process. In practice, to avoid this problem, filters of the dead-end type are often designed multiple times in parallel to enable continuous processing. In backwashing, the pressure across the filter is periodically inverted, so that permeate flows back into the feed, lifting the particles layer from the surface of the filter.

Crossflow filtration (also known as tangential flow filtration) is a technique in which the solution to be purified flows parallel along the filter medium. The filtrate passes the filter medium without or almost without causing a filter cake to be formed because the solid particles are swept off the filter medium by the flow of the solution to be purified. As no filter cake or almost no filter cake is formed the length of time that a filter unit can be operational is increased. Crossflow filtration can be both a continuous process or a batch process, unlike batch-wise dead-end filtration. Optionally, the catalyst particles accumulated on the cross-filter are removed by backwashing.

Optionally, downstream of the hydrogenation catalyst particles filtration section are one or more guard filters installed. In general guard filters are filters based on dead-end filtration and have a small pore size. Their main purpose is to catch particles that are slipped through the main filter(s) during normal operation or to catch particles in case of breakage or leakage of the main filter.

In a preferred embodiment of the loop venturi reaction device of the invention, the filtration device is inserted into the external circulation loop and is a cross-flow filtration unit.

The loop venturi reaction device may comprise additional modules, before, between or after the above-listed modules. For instance, the device may further comprise a measuring apparatus for pressure, temperature, flow and concentrations. Preferably, inline measurement equipment is installed.

Preferably, the loop venturi reaction device comprises a device for charging catalyst and/or activator to the buffered aqueous phosphoric acid-containing solution. Preferably, the loop venturi reaction device comprises a batch-wise operated device for charging catalyst and/or activator to the buffered aqueous phosphoric acid-containing solution. Preferably, this device comprises a vessel and a stirrer that are inertized or kept under hydrogen gas during operation. In a preferred embodiment of the process according to the invention, the loop venturi reaction device further comprises a batch-wise operated device for charging catalyst and/or activator to the buffered aqueous phosphoric acid containing solution.

The material of construction of the loop venturi reaction device of the invention can comprise steel, because of its high tensile strength. A problem with using nitric acid-containing reaction liquid is that it tends to dissolve the steel of the loop venturi reaction device where it is used in. Most steels contain some molybdenum. It is known that molybdenum is detrimental to the selectivity of the hydroxylamine reaction, for example 1 ppm of Mo in the reaction medium can lead to a more than 2 % reduction in hydroxylamine selectivity.

As used herein, 'hydroxylamine selectivity' (the selectivity towards the production of hydroxylamine) is defined as follows: molar ratio of the amount of hydroxylamine produced in the loop venturi reaction device divided by half the amount of H⁺ consumed in the loop venturi reaction device. A low selectivity means that more by-products are generated which is not desirable.

The activity of the catalyst is defined herein as grams hydroxylamine produced per gram catalyst per hour. In other words, activity is related to the rate of production of hydroxylamine.

A range of steels are well known for the manufacture of vessels, pipes and reactors for the preparation of hydroxylamine. This includes steels known as 304, 316, 304L or 316L. Different grades have differing levels of molybdenum and other metals. Furthermore, the level of carbon and other elements can also impact corrosion resistance.

The carbon ranges are 0.08 wt% maximum for grades 304 and 316 and 0.030 wt% maximum for the 304L and 316L grades. All other element ranges are essentially the same (for example the nickel range for 304 is 8.00 to 10.50 wt% and for 304L is 8.00 to 12.00 wt%).

The lower carbon 'variants' (316L) were established as alternatives to the 'standard' (316) carbon range grade to overcome the risk of intercrystalline corrosion (weld decay), which was identified as a problem in the early days of the application of these steels. This can result if the steel is held in a temperature range 450 to 850°C for periods of several minutes, depending on the temperature and subsequently exposed to aggressive corrosive environments. Corrosion then takes place next to grain boundaries.

Preferably the carbon level of the steel is 0 to 0.03 wt% C. If the carbon level is below 0.030 wt% then this intercrystalline corrosion does not take place following exposure to these temperatures, especially for the sort of times normally experienced in the heat affected zone of welds in 'thick' sections of steel. Low carbon types may also be easier to weld than the standard carbon types.

Steel may also be annealed. Annealing is a heat treatment wherein a material is altered, causing changes in its properties such as strength and hardness. It is a process that produces conditions by heating to above the recrystallization temperature, maintaining a suitable temperature, and then cooling. In the cases of steel this process is performed by substantially heating the material (generally until glowing) for a while and allowing it to cool. Annealing does not reduce the carbon content but makes the distribution of element more homogenous and therefore improves corrosion resistance.

Even more preferably austenite steel is used. Austenite, also known as gamma phase iron, is a metallic non-magnetic allotrope of iron or a solid solution of iron, with an alloying element. In plain-carbon steel, austenite exists above the critical eutectoid temperature of circa 1,000 K; other alloys of steel have different eutectoid temperatures.

According to a preferred embodiment of the invention the walls of the loop venturi reaction device comprise steel comprising 0 to 0.08 wt% C and 0 to 0.03 wt% Mo. According to a particularly preferred embodiment of the invention the walls of the loop venturi reaction device comprise steel and the steel is selected from the group consisting of:
- quench annealed steel A comprising 0 to 0.08 wt% C, 0 to 2.0 wt% Mn, 0 to 2.0 wt% Si, 0 to 0.045 wt% P, 0 to 0.03 wt% S, 17 to 21 wt% Cr, 0 to 0.03 wt% Mo, 8.0 to 13 wt% Ni, the remainder being Fe and unavoidable impurities;
- low carbon steel B comprising 0 - 0.03 wt% C, 0 to 2.0 wt% Mn, 0 to 1.0 wt% Si, 0 to 0.045 wt% P, 0 to 0.03 wt% S, 17 to 21 wt% Cr, 0 to 0.03 wt% Mo, 8.0 to 13.0 wt% Ni, the remainder being Fe and unavoidable impurities;
- stabilized Steel C comprising 0 to 0.08 wt% C, 0 to 2.0 wt% Mn, 0 to 2.0 wt% Si, 0 to 0.045 wt% P, 0 to 0.04 wt% S, 17 to 21 wt% Cr, 0 to 0.03 wt% Mo, 9.0 to 13.0 wt% Ni and either Ti (minimum: 5 times wt% C (carbon) to maximum: 0.8 wt%) or Nb + Ta (minimum: 8 times wt% C (carbon) to maximum: 1.1 wt%), the remainder being Fe and unavoidable impurities.

Using such a steel with limited Mo-content further improves the selectivity of the hydrogenation reaction towards hydroxylamine.

The loop venturi device of the present invention is configured to carry out an industrial scale process and is an industrial scale device. By "industrial scale" is meant a production capacity, or a device capable of operating at a production capacity for hydroxylamine of at least 100 tons per annum if operated all the time. In a preferred embodiment, the device according to the invention has a production capacity for hydroxylamine of at least 1000 tons per annum, preferably at least 25,000 tons per annum, more preferably at least 50,000 tons per annum, even more preferably at least 75,000 tons per annum and most preferably at least 100,000 tons per annum.

Finally, a further object of the invention is the use the loop venturi reaction device of the invention as stand-alone unit for the production of hydroxylamine or as a part of a chemical plant for the production of oximes, preferably as part of a chemical plant for the production of oximes, more preferably as part of a chemical plant for the production of butanone oxime, cyclopentanone oxime, cyclohexanone oxime and/or cyclodedocecanone oxime, most preferably as part of a chemical plant for the production of cyclohexanone oxime.

The use as stand-alone unit has the advantage that the hydroxylamine in the hydroxylamine-containing buffered aqueous phosphoric acid-containing solution can be directly used for various chemical reactions without complex and expensive separation and purification steps. The advantage of being part of a chemical plant for the production of oximes is that the buffered aqueous phosphoric acid-containing solution that is formed after the hydroxylamine in the hydroxylamine-containing buffered aqueous phosphoric acid solution has reacted away can be reused, preferably after purification and addition of nitrate in the process according to the invention. This is beneficial because valuable compounds, especially nitrate and phosphate, present in the hydroxylamine in the hydroxylamine-containing buffered aqueous phosphoric acid solution are not lost.

### BRIEF DESCRIPTION OF THE DRAWINGS

In the following, the invention and prior art will be described with reference to the FIGURES 1 -5.
- **FIGURE 1**: schematic diagram of a device suitable for producing oxime according to the HPO^{®} and HPO^{®plus} technologies for the production of oximes.
- **FIGURE 2**: schematic diagram of a device suitable for producing hydroxylamine as part of a device suitable for producing oxime according to the HPO^{®} and HPO^{®plus} technologies for the production of oximes according to prior art.
- **FIGURE 3**: schematic diagram of the structure of an example of a gas-suspension ejector of the present invention.
- **FIGURE 4**: schematic diagram of a loop venturi reaction device suitable for producing hydroxylamine according to the invention as part of a device suitable for producing oxime according to the HPO^{®} and HPO^{®plus} technologies for the production of oximes.
- **FIGURE 5**: schematic diagram of an alternative loop venturi reaction device suitable for producing hydroxylamine according to the invention as part of a device suitable for producing oxime according to the HPO^{®} and HPO^{®plus} technologies for the production of oximes.

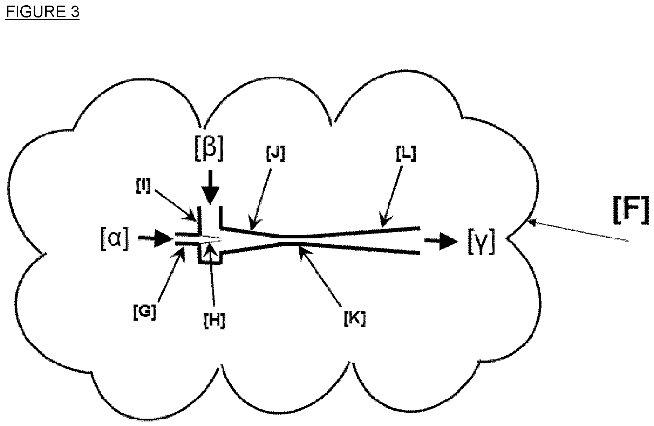

The invention, however, is as defined in the claims and as generally described herein. It should not be limited to the embodiments shown for illustrative purposes in Figures 3 to 5 below.

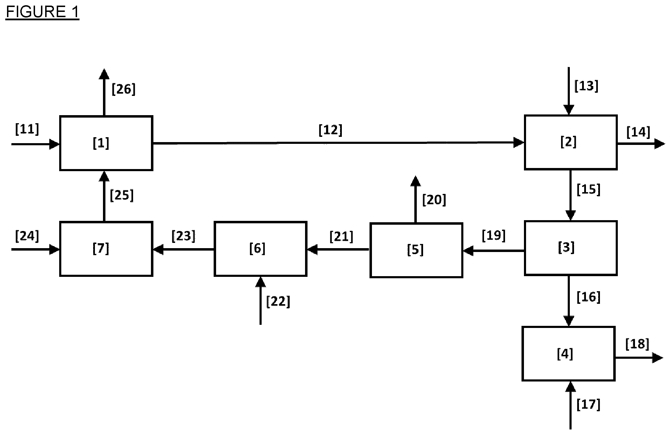

FIGURE 1 represents a schematic diagram of a device suitable for producing oxime according to the HPO^{®} and HPO^{®plus} technologies for the production of oximes. Hydrogen-containing gas is provided through line [11] to hydroxylamine formation zone [1]. A nitrate-containing buffered aqueous phosphoric acid-containing solution is provided via line [25]. A gas mixture containing hydrogen and non-hydrogen compounds like N₂O is discharged through line [26]. A hydroxylamine-containing buffered aqueous phosphoric acid-containing solution is provided via line [12] to hydroxylamine removal zone [2], also known as an oximation section. Ketone is provided through line [13]. After reaction in the oximation section, the resulting oxime is removed in an organic phase through line [14]. A buffered aqueous phosphoric acid containing solution is passed through line [15] to separating unit [3]. Periodically a portion of the buffered aqueous phosphoric acid containing solution is removed via line [16] to neutralization section [4]. Ammonia supplied via line [17] is used for neutralization. The resulting neutralized solution is removed via line [18]. The remaining buffered aqueous phosphoric acid containing solution is passed via line [19] to water removal zone [6], where the solution is concentrated by removal of water. The removed water is discharged via line [20]. The resulting concentrated buffered aqueous phosphoric acid containing solution is passed via line [21] to ammonia conversion zone [6], where nitrous gases supplied via line [22] are used to convert ammonia, therein. The obtained buffered aqueous phosphoric acid containing solution with reduced ammonia content passes via line [23] to addition unit [7] where nitrous gases and/or nitric acid are added via line [24] and dissolved in the solution. The resulting nitrate containing buffered aqueous phosphoric acid containing solution is fed via line [25] to hydroxylamine formation zone [1] and the process continues in a loop.

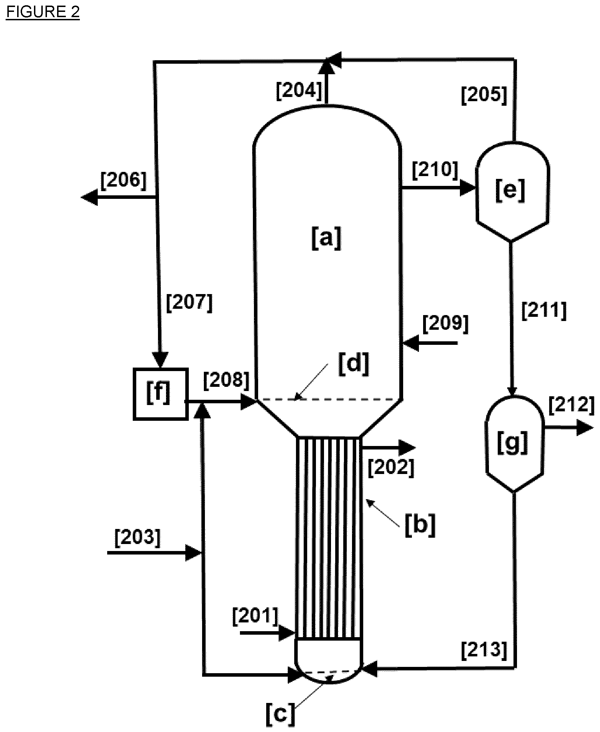

FIGURE 2 is a schematic diagram of a device suitable for producing hydroxylamine as part of a device suitable for producing oxime according to the HPO^{®} and HPO^{®plus} technologies for the production of oximes according to the prior art. It comprises an autoclave 3-phase sparged bubble column reactor [a], a cooling element [b], a first gas distributor [c], a second gas distributor [d], gas-suspension separators [e], a gas recycle compressor [f], and catalyst filtration units [g]. The autoclave 3-phase sparged bubble column reactor [a] is positioned vertically and is equipped with a water-cooled shell-and-tube type heat exchanger [b] to remove heat of hydrogenation reaction. The cooling water enters the heat exchanger [b] via line [201] and is, after absorbing heat, discharged via line [202]. The autoclave 3-phase sparged bubble column reactor [a] is further equipped with a first gas distributor [c] for producing hydrogen rich gas bubbles below heat exchanger [b] and with a second gas distributor [d] for producing hydrogen rich gas bubbles in the lower part of the main body of bubble column reactor [a]. Fresh hydrogen-containing gas is provided via line [203] to the hydrogenation device. A part of the fresh hydrogen-containing gas is charged to the reactor through the first gas distributor [c], while the remaining part is charged to the reactor through the second gas distributor [d]. A gas mixture containing hydrogen and non-hydrogen compounds like N₂O is discharged from the top of the bubble column reactor [a] through line [204]. This gas mixture and the gas mixture discharged via one of the lines [205] (only one is shown in the Figure) from one of the gas-suspension separators [e] (only one is shown in the Figure) are combined. A part of the resulting combined gas mixture is discharged via line [206] from the device. The remainder of the gas mixture is charged via line [207] to a gas recycle compressor [f]. The gas mixture discharged from gas recycle compressor [f] and part of the fresh hydrogen-containing gas charged via line [203] are combined and charged to the reactor via line [208]. The gas mixture is dispersed in the buffered aqueous phosphoric acid reaction mixture via the second gas distributor [d]. A nitrate-containing buffered aqueous phosphoric acid-containing solution is provided via line [209]. The nitrate-containing buffered aqueous phosphoric acid-containing solution is obtained by charging fresh nitric acid or nitrogen oxides to a buffered aqueous phosphoric acid-containing solution (not shown in the Figure). A multi-phase mixture containing hydroxylamine-containing buffered aqueous phosphoric acid-containing solution, hydrogenation catalyst particles and hydrogen-containing gas flows via one of the lines [210] (only one is shown in the Figure) into the gas-suspension separators [e]. In the gas-suspension separators [e] a gas phase is separated from the multi-phase mixture and is discharged via one of the lines [205] (only one is shown in the Figure). The remainder of the multi-phase mixture is discharged through the bottom of the gas-suspension separators [e] via the lines [211] (only one is shown in the Figure) and is then charged into one of the catalyst filtration units [g] (only one is shown in the Figure). In the catalyst filtration units [g] hydroxylamine containing buffered aqueous phosphoric acid containing solutions that are virtually free of catalyst particles are separated and discharged via one of the lines [212] (only one is shown in the Figure) to the oximation section (not shown in the Figure). Mixtures comprising hydroxylamine containing buffered aqueous phosphoric acid containing solutions and catalyst particles are discharged from the catalyst filtration units [g] via the lines [213] (only one is shown in the Figure) and then charged to the bottom of bubble column reactor [a].

FIGURE 3 is a schematic diagram of the structure of an example of a gas-suspension ejector [F] of the present invention. The gas-suspension ejector [F] comprises a suspension inlet [G], a fluid nozzle [H], a suction chamber [I], a convergent inlet nozzle [J], a throat [K], and a divergent outlet diffuser [L]. A suspension of hydroxylamine-containing buffered aqueous phosphoric acid-containing solution and catalyst particles [a] goes into the gas-suspension ejector [F] via suspension inlet [G]. Preferably, the flow [a] entering the gas-suspension ejector [F] also contains gas bubbles. The suspension and preferably gas bubbles then enters the suction chamber [I] via the fluid nozzle [H]. In the suction chamber [I] fresh hydrogen containing gas [β] is introduced via inlet [M]. Where fresh hydrogen-containing gas is defined as hydrogen-containing gas originating from outside the loop venturi reaction device [X]. Fresh hydrogen-containing gas can originate from different sources and be produced by different process, for example through steam methane reforming, electrolysis of water, methanol dehydrogenation, (brown) coal gasification, biomass gasification, methane pyrolysis, cracking of hydrocarbon feedstock and extraction of underground hydrogen. In addition, fresh hydrogen-containing gas can also be made by removing non-hydrogen compounds from the hydrogen- and N₂O-containing gas discharged from a loop venturi reaction device [X]. In addition to fresh hydrogen-containing gas, hydrogen-containing gas accumulating in the upper part of the reaction vessel [Z] can also be fed into suction chamber [I] (not shown in the Figure). The suspension flow drags gas from the suction chamber [I] to the convergent inlet nozzle [J] and then through throat [K] to the divergent outlet diffuser [L], creating a dispersion of very fine hydrogen-containing gas bubbles in the suspension of hydroxylamine-containing buffered aqueous phosphoric acid containing solution and catalyst particles. The obtained dispersion [γ] is discharged from the gas-suspension ejector [F] and charged into the reaction vessel [Z] (not shown in the Figure).

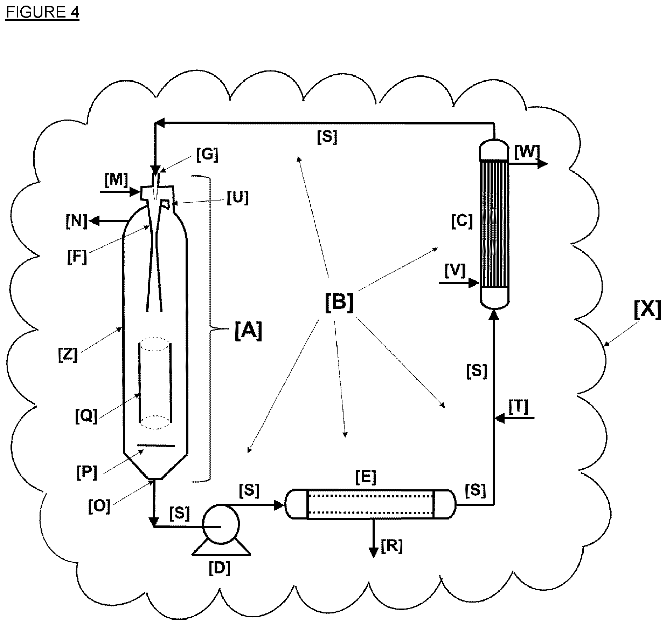

FIGURE 4 is a schematic diagram of a loop venturi reaction device [X] suitable for producing hydroxylamine according to the invention. The loop venturi reaction device [X] comprises a vertically upright hydrogenation reactor [A] with an height to width ratio of at least 2, an external circulation loop [B], a water-cooled indirect heat exchanger [C], a circulation pump [D], a catalyst filtration device [E], a downward-directed gas-suspension ejector [F], a suspension inlet [G], inlet of fresh hydrogen containing gas [M], a gas discharge line [N], a suspension outlet [O], a diverter [P], a circulation tube [Q], an outlet of hydroxylamine containing buffered aqueous phosphoric acid containing solution [R], equipment connecting line segments [S], an inlet of nitrate containing buffered aqueous phosphoric acid containing solution [T], gas recycle line [U], inlet cooling water [V], outlet cooling water [W], and reaction vessel [Z]. The autoclave hydrogenation reactor [A] is positioned vertically and is equipped with a downward-directed gas-suspension ejector [F] mounted in the top of the reaction vessel [Z]. The gas suction chamber of gas-suspension ejector [F] is located outside the reaction vessel [Z]. This has the advantage that the line transporting fresh hydrogen gas can easily be connected to and disconnected from gas inlet [M]. Gas recycle line [U] connects the upper part of the reaction vessel [Z] and the gas suction chamber of gas-suspension ejector [F]. Hydrogen-containing gas flows via gas recycle line [U] into the gas suction chamber of gas-suspension ejector [F]. A suspension of hydroxylamine-containing buffered aqueous phosphoric acid containing solution and Pd-containing hydrogenation catalyst particles enters downward directed gas-suspension ejector [F] via suspension inlet [G]. The reaction mixture sprayed into the upper region of the reaction vessel [Z] and then flows into a central inserted a circulation tube [Q], which is arranged in the longitudinal direction of the reactor.

Optionally, reactor [A] is not equipped with a circulation tube [Q] (not shown in the Figure). A diverter [P] is arranged below the ejector in the lower region of the reaction vessel [Z]. Both the circulation tube [Q] and the diverter [P] improve the mixing of the reaction mixture in the reaction vessel [Z]. Optionally, reactor [A] is not equipped with a diverter [P] (not shown in the Figure). The outlet of the gas-suspension ejector [F] through which the reaction mixture is sprayed into the upper region of the reaction vessel [Z] can be either above or below the surface of the reaction mixture, i.e., a suspension of a hydroxylamine-containing buffered aqueous phosphoric acid containing solution and Pd-containing hydrogenation catalyst particles, and hydrogen-containing gas bubbles, in the reaction vessel [Z]. Optionally, the reaction vessel [Z] is equipped with more than one downward-directed gas-suspension ejector [F] (not shown in the Figure). This has the advantage that the length of the jet nozzle can be reduced compared to one single jet nozzle. A gas mixture containing hydrogen and inerts like N₂O is discharged from reactor [A] via gas discharge line [N]. Optionally, the gas mixture that is discharged via gas discharge line [N] is purified to remove at least part of the inerts and subsequently at least part of the hydrogen containing discharged gas is recharged to reactor [A], optionally via inlet of fresh hydrogen containing gas [M] (not shown in the Figure). A suspension of hydroxylamine-containing buffered aqueous phosphoric acid-containing solution and hydrogenation catalyst particles, and in general also hydrogen-containing gas bubbles is discharged from the reactor [A] via suspension outlet [O] to the external circulation loop [B]. The outlet can be located at any desired point of the reactor [A], but preferably at the lower part of the reactor [A]. The external circulation loop [B] connects the suspension outlet [O] of the reactor [A] with the suspension inlet [G] of gas-suspension ejector [F]. In the external circulation loop [B] first a circulation pump [D], then a catalyst filtration device [E] and subsequently a water-cooled indirect heat exchanger [C], are inserted. The various pieces of equipment are connected via equipment connecting line segments [S]. The order of the circulation pump [D], the catalyst filtration device [E] and the water-cooled indirect heat exchanger [C] in the external circulation loop [B] may differ from what is shown in FIGURE 3. For instance, the order may first be the circulation pump [D], then the water-cooled indirect heat exchanger [C] and subsequently the catalyst filtration device [E]. The circulation pump [D] circulates the hydroxylamine-containing buffered aqueous phosphoric acid-containing solution in the loop venturi reaction device [X]. The circulation rate of the hydroxylamine-containing buffered aqueous phosphoric acid-containing solution in the loop venturi reaction device [X] can be varied over a wide range. In general, the circulation rate of the hydroxylamine-containing buffered aqueous phosphoric acid-containing solution is varied between 10 and 500 times per hour, preferably between 15 and 300 times per hour, most preferably between 20 and 250 times per hour. Where the circulation rate (expressed in times per hour) is defined as the flow rate of the hydroxylamine-containing buffered aqueous phosphoric acid-containing solution through the external circulation loop (expressed in m³/hr) divided by the total volume of the hydroxylamine- containing buffered aqueous phosphoric acid-containing solution in the loop venturi reaction device [X] (expressed in m³). In a preferred embodiment of the process according to the inventions, the circulation rate of the buffered aqueous phosphoric acid-containing solution is ranging from 10 and 500 times per hour, preferably from 15 to 300 times per hour.

In the catalyst filtration device [E] a hydroxylamine-containing buffered aqueous phosphoric acid-containing solution that is virtually free of catalyst particles [R] is separated of. The catalyst filtration device [E] may be any type of catalyst filtration device. Preferably, catalyst filtration device applies cross-flow filtration technology or dead-end filtration technology, most preferably cross-flow filtration technology. Optionally, flow [R] is further filtered to remove the remaining catalyst particles in a downstream located second filtration unit (not shown in the Figure). The loop venturi reaction device [X] is equipped with a water-cooled shell-and-tube type heat exchanger [C] to remove heat of the hydrogenation reaction. The cooling water enters the heat exchanger [C] via line [V] and is, after absorbing heat, discharged via line [W]. A nitrate-containing buffered aqueous phosphoric acid-containing solution is supplied via line [T] to the external circulation loop [B] downflow of the catalyst filtration device [E]. Alternatively, the nitrate-containing buffered aqueous phosphoric acid-containing solution is provided via line [T] to another location of the loop venturi reaction device [X], for example to the reaction vessel [Z]. The nitrate-containing buffered aqueous phosphoric acid-containing solution is obtained by charging fresh nitric acid or nitrogen oxides to a buffered aqueous phosphoric acid-containing solution (not shown in the Figure).

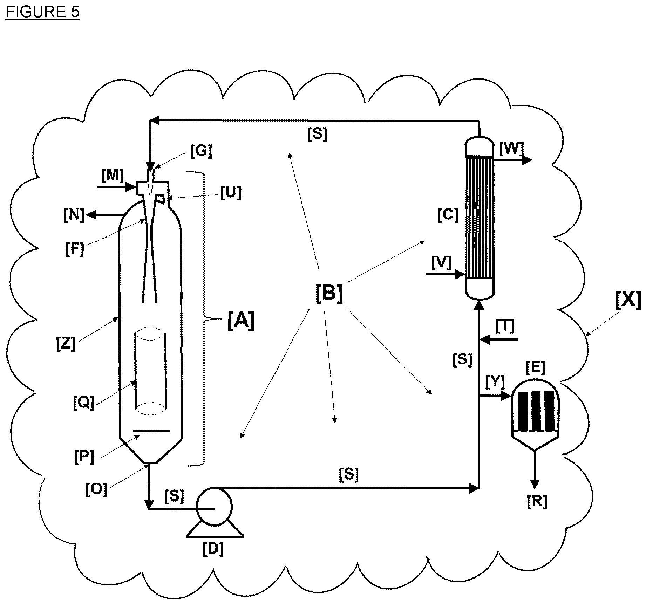

FIGURE 5 is a schematic diagram of an alternative loop venturi reaction device [X] suitable for producing hydroxylamine according to the invention. The vertically upright hydrogenation reactor [A] with an height to width ratio of at least 2 and the external circulation loop [B] are essentially analogous to the vertically upright hydrogenation reactor [A] with an height to width ratio of at least 2 and the external circulation loop [B] that is illustrated in FIGURE 4, except that the catalyst filtration device [E] is not inserted into the external circulation loop [B], but is inserted into a line [Y] that branches off from the external circulation loop [B]. In the catalyst filtration device [E] a hydroxylamine-containing buffered aqueous phosphoric acid-containing solution that is virtually free of Pd-containing hydrogenation catalyst particles [R] is separated. The catalyst filtration device [E] may be any type of catalyst filtration device. Preferably, catalyst filtration device uses cross-flow filtration technology or dead-end filtration technology, most preferably dead-end filtration technology. Optionally, flow [R] is further filtered to remove the remaining catalyst particles in a downstream located second filtration unit (not shown in the Figure). Optionally, a stream containing Pd-containing hydrogenation catalyst particles or a stream containing a suspension of Pd-containing hydrogenation catalyst particles in an aqueous solution, for example a buffered aqueous phosphoric acid-containing solution, is discharged from the catalyst filtration device [E] and charged to any desired point in the loop venturi reaction device [X].

While the present invention has been illustrated by means of several preferred embodiments, one of ordinary skill in the art will recognize that modifications, equivalent substitutions, improvements, etc. can be made while still remaining within the scope and spirit of the present invention. The present invention can be operated at industrial scale. The device suitable for producing hydroxylamine can be operated in a continuous mode. Accordingly, no limitation upon the invention is intended, except as set forth in the appended claims.

The invention will also be described in further detail in the following examples, which are not intended to limit the scope of this invention, as defined by the attached claims.

### EXAMPLES

### COMPARATIVE EXAMPLE 1

This comparative example was carried out in a commercial nitrate hydrogenation device that was part of a HPO^{®plus} plant for the production of cyclohexanone oxime, which was downstream converted into ε-caprolactam. The HPO^{®plus} plant substantially corresponds to that of FIGURE 1 and the nitrate hydrogenation device substantially corresponds to that of FIGURE 2. Over a number of years, large amounts of data have been collected on this nitrate hydrogenation device. During this period, the actual hydroxylamine production rate from the nitrate hydrogenation device was not constant but varied mainly due to market conditions. The production rates of hydroxylamine, converted to annual figures, varied between approximately 25,000 tons per annum and approximately 50,000 tons per annum. The catalyst applied for the selective reduction of nitrate was 10% Pd/C. The nitrate concentration of the nitrate containing buffered aqueous phosphoric acid containing solution feed of the nitrate hydrogenation device varied from 2 to 3 mol/kg buffered aqueous phosphoric acid containing solution. The nitrate concentration of the hydroxylamine containing buffered aqueous phosphoric acid containing solution discharged from the nitrate hydrogenation device varied from 0.5 to 1.0 mol/kg buffered aqueous phosphoric acid containing solution. The acid concentration of the hydroxylamine containing buffered aqueous phosphoric acid containing solution discharged from the nitrate hydrogenation device varied from 0.4 to 0.6 mol/kg buffered aqueous phosphoric acid containing solution. The phosphate concentration of the hydroxylamine containing buffered aqueous phosphoric acid containing solution discharged from the nitrate hydrogenation device varied from 2.8 to 3.8 mol/kg buffered aqueous phosphoric acid containing solution. Tthe hydroxylamine concentration of the hydroxylamine containing buffered aqueous phosphoric acid containing solution discharged from the nitrate hydrogenation device was always kept about 1 mol/kg of buffered aqueous phosphate solution.

During this time, the hydrogen partial pressure of the gas flow entering the 3-phase bubble column via the gas distributor above the heat exchanger was varied between 10 and 20 bar, while the partial hydrogen pressure of the fresh hydrogen was maintained at approximately 25 bar. The coolant used in the heat exchanger under the main body of the bubble column was cooling water extracted from the cooling water grid. During this time, the average temperature of the buffered aqueous phosphoric acid containing solution in the 3-phase bubble column ranged from 42 to 50 °C. And during this time, the selectivity towards hydroxylamine varied from 82 to 89 %.

### COMPARATIVE EXAMPLE 2

The performance of the above system was translated into a numerical model developed in GNU Octave version 7.1.0, which described the system using a combination of hydrogen gas-liquid mass transfer, hydrogen liquid-solid mass transfer, reaction kinetics, mass and heat balances. The outcome of the simulations with this program included production rates and selectivity values towards desired hydroxylamine and undesired by-products. The model predicted the impact of many variables including temperature, partial hydrogen pressure, nitrate content, gas hold-up, superficial gas velocity, liquid residence time, catalyst hold-up and catalyst particle size. The description of reaction kinetics was obtained from lab scale experiments under representative conditions and by using an extended version of equations as given in Chem.-Ing.-Tech. 59 (1987) Nr. I, p. 72-73 and in Chem.-Ing.-Tech. 60 (1988) Nr. 4, p. 302-304 by A. Schumpe et al. The description of the nitrate hydrogenation device was calibrated on the basis of production data from the commercial operating hydrogenation device described in COMPARATIVE EXAMPLE 1. The model was adapted to simulate the performance of nitrate hydrogenation reactions in loop venturi reaction devices. The performance of these alternative devices was characterized by the production rates and selectivity values towards both the desired hydroxylamine and the undesired by-products. The outcome of these simulations was compared to the performance of the commercial nitrate hydrogenation device described in COMPARATIVE EXAMPLE 1.

### EXAMPLE 1: Impact of enhanced heat transfer

This example was carried out in equipment substantially similar to that of FIGURE 3.

The simulation model described in COMPARATIVE EXAMPLE 2 that was developed to describe the process shown in COMPARATIVE EXAMPLE 1 was adapted for the equipment configuration as depicted in FIGURE 3. Almost all parameters were the same as those describing the prior art device in COMPARATIVE EXAMPLE 1, including total liquid hold-up in the device, partial hydrogen pressure in the device, hydrogen gas-liquid mass transfer, hydrogen liquid-solid mass transfer, catalyst concentration, average liquid residence time in the device, nitrate concentration in the nitrate containing buffered aqueous phosphoric acid containing solution feed of the nitrate hydrogenation device, and dimensions of the heat exchanger. The type of coolant, cooling water extracted from the cooling water network, temperature of the coolant and the feed rate of coolant charged to the heat exchanger were fixed values in the comparisons of both devices. The main difference between the simulations of both devices was the liquid flow rate on the product side of the heat exchangers. In case of the loop venturi reactor device with the heat exchanger in the external circulation loop much higher liquid flow rates (product side) could be obtained compared to the traditional device with the heat exchanger below the main body of the bubble column.

The simulations showed that under a range of process conditions, the average temperature of the hydroxylamine containing buffered aqueous phosphoric acid containing solution in the loop venturi device could easily be reduced by several degrees Celsius compared to the traditional device. The main consequence of this reduced temperature is an improved selectivity towards the desired hydroxylamine up to approximately 2 %, while the selectivity towards the undesired by-products was reduced by the same percentage.

### EXAMPLE 2: Impact of improved gas-liquid mass transfer

This example was carried out in equipment substantially similar to that of FIGURE 3.

The simulation model described in COMPARATIVE EXAMPLE 2 and developed for COMPARATIVE EXAMPLE 1 was adapted for the equipment configuration as depicted in FIGURE 3. Almost all parameters, were taken in the same way as those describing the prior art device in COMPARATIVE EXAMPLE 1, including total liquid hold-up in the device, partial hydrogen pressure in the device, hydrogen liquid-solid mass transfer, catalyst concentration, nitrate concentration in the nitrate containing buffered aqueous phosphoric acid containing solution feed of the nitrate hydrogenation device, and the heat transfer rate of the heat exchanger.

The main difference between the simulations of both devices was the hydrogen gas-liquid mass transfer rate due to usage of different types of equipment. In the case of the loop venturi reactor device, a venturi jet type of gas disperser was applied creating much smaller hydrogen containing gas bubbles compared to the traditional device with sparger type gas distributors.

The simulations showed under a wide range of process conditions, the hydrogen gas-liquid mass transfer rates in the loop venturi device were enhanced compared to the traditional device. The most important consequences of this enhanced gas-liquid mass transfer were both an improved selectivity towards the desired hydroxylamine up to approximately 2 %, while the selectivity towards the undesired by-products was reduced by the same percentage and an increased production rate of hydroxylamine up to approximately 30%.

EXAMPLE 1 shows that in a loop venturi reactor device according to the invention a reduction of the average temperature of the reaction liquid can be achieved and as a consequence the selectivity of the hydrogenation of nitrate towards hydroxylamine can be significantly improved compared with the prior art process.

EXAMPLE 2 shows that in a loop venturi reactor device according to the invention an increased hydrogen gas-liquid mass transfer can be achieved and as a consequence both the selectivity of the hydrogenation of nitrate towards hydroxylamine and the production rate of hydroxylamine can be significantly improved compared with the prior art process.

These examples show that by improving heat transfer and/or hydrogen gas-liquid mass transfer during the process of nitrate hydrogenation in buffered aqueous phosphoric acid containing solutions as a whole results in a higher selectivity towards hydroxylamine. This results in lower consumption figures of the raw materials nitrate and hydrogen for the production of hydroxylamine, reducing the variable costs of hydroxylamine production and improving the carbon footprint of hydroxylamine. Furthermore, due to a reduced amount of by-products formed, the costs associated with the elimination of these by-products were reduced.

In addition, improving the hydrogen gas-liquid mass transfer during the process of nitrate hydrogenation in buffered aqueous phosphoric acid containing solutions is shown to increase the production rate of hydroxylamine. This results in an overall nitrate hydrogenation process that has higher production of hydroxylamine, reducing the fixed costs of the production of hydroxylamine, i.e., the costs are spread over a larger amount of hydroxylamine produced.

Accordingly, EXAMPLES 1 and 2 demonstrate a major improvement over the prior art process.

## Claims

1. A continuous process for preparing hydroxylamine in a buffered aqueous phosphoric acid-containing solution by hydrogenating nitrate with hydrogen at a pressure above atmospheric pressure in the presence of suspended Pd-containing hydrogenation catalyst particles in a loop venturi reaction device ([X]) comprising a hydrogenation reactor ([A]) and an external circulation loop ([B]), wherein the hydrogenation reactor ([A]) comprises a downward-directed gas-suspension ejector ([F]) in the upper region of the hydrogenation reactor ([A]), comprising a reaction mixture inlet ([G]), a motive fluid nozzle ([H]), a suction chamber ([I]), a throat ([K]), a divergent outlet diffuser ([L]), and optionally, a convergent inlet nozzle ([J]), wherein the process comprises the steps of:
a) charging a fresh nitrate-containing buffered aqueous phosphoric acid-containing solution into the hydrogenation reactor ([A]) and/or into the external circulation loop ([B]);
b) charging fresh hydrogen-containing gas into the loop venturi reaction device ([X]), preferably into the hydrogenation reactor ([A]);
c) hydrogenating nitrate charged in step a) with hydrogen charged in step b) under the influence of Pd-containing hydrogenation catalyst particles, wherein the hydrogenating is carried out at temperatures ranging from 20 to 65 °C, preferably from 25 to 55 °C, most preferably from 30 to 45 °C, to provide a reaction mixture comprising hydroxylamine-containing buffered aqueous phosphoric acid-containing solution, hydrogenation catalyst particles and hydrogen-containing gas;
d) circulating the reaction mixture through the loop venturi reaction device ([X]);
e) cooling of the reaction mixture, wherein the cooling is performed with water in an indirect heat exchanger ([C]) located in the external circulation loop ([B]);
f) discharging a hydroxylamine-containing buffered aqueous phosphoric acid containing solution from the loop venturi reaction device ([X]), wherein the reaction mixture is filtered in a filtration device ([E]) inserted into the external circulation loop ([B]) or is passed through a line ([Y]) branching off from the external circulation loop ([B]) connecting the external circulation loop ([B]) to a filtration device ([E]) prior to separate part of the hydroxylamine-containing buffered aqueous phosphoric acid-containing solution from the reaction mixture;
g) discharging a N₂O-containing gas from the loop venturi reaction device ([X]); wherein
- the gas holdup of the reaction mixture that flows through the external circulation loop ([B]) is at least 5 vol. %, preferably at least 8 vol. %; more preferably at least 12 vol. % and most preferably at least 15 vol. %,
- the phosphate concentration as determined in the hydroxylamine-containing buffered aqueous phosphoric acid containing solution discharged from the loop venturi reaction device in step f) is from 2 to 4 moles/kg, preferably from 2.8 to 3.8 moles/kg, and
- the acid concentration in the buffered aqueous phosphoric acid-containing solution is from 0.3 to 0.7 moles/kg, preferably from 0.4 to 0.6 moles/kg.

2. The process according to claim 1, wherein the circulation rate of the reaction mixture is ranging from 10 and 500 times per hour, preferably from 15 to 300 times per hour.

3. The process according to claim 1 or 2, wherein the inert gas partial pressure in the hydrogenation reactor ([A]) ranges from 0.5 to 50 bar, preferably from 2 to 40 bar, most preferably from 5 to 30 bar.

4. The process according to any of the preceding claims, wherein the ratio of the nitrate concentration expressed in mol/kg of the hydroxylamine-containing buffered aqueous phosphoric acid-containing solution discharged from the loop venturi reaction device ([X]) in step f) and the nitrate concentration expressed in mol/kg of the nitrate-containing buffered aqueous phosphoric acid-containing solution charged into the loop venturi reaction device [X] in step a) ranges from 1:2 to 1:6, more preferably from 1:2.5 to 1:5.

5. The process according to any of the preceding claims, wherein the charging of Pd-containing hydrogenation catalyst particles into the hydrogenation reactor ([A]) and/or the external circulation loop ([B]) is performed in a batch-wise mode.

6. The process according to any of the preceding claims, wherein the N₂O concentration of the N₂O-containing gas that is discharged in step g) is ranging from 0 to 2 vol. %, in particular at from 0.01 to 1 vol. %, more in particular at from 0.05 to 0.5 vol. %, most particularly at from 0.08 to 0.3 vol. %.

7. The process according to any of the preceding claims, wherein the hydroxylamine concentration in the hydroxylamine-containing buffered aqueous phosphoric acid-containing solution discharged from the loop venturi reaction device ([X]) in step f) is ranging from 0.50 to 2 mol/kg, more preferably is about 1 mol/kg of buffered aqueous phosphate acid-containing solution.

8. The process according to any of the preceding claims, wherein the N₂O-containing gas that is discharged in step g) is purified and at least part of the discharged N₂O-containing gas is re-used for the hydrogenation reaction, preferably wherein the purification is performed by adsorption or membrane separation, whereby at least part of the N₂O is removed from the N₂O-containing gas.

9. A loop venturi reaction device ([X]) for continuously preparing hydroxylamine in a buffered aqueous phosphoric acid containing solution by hydrogenating nitrate with hydrogen at a pressure above atmospheric pressure in the presence of suspended Pd-containing hydrogenation catalyst particles, wherein the loop venturi reaction device ([X]) comprises
(a) a hydrogenation reactor ([A]), preferably having a height to width ratio of at least 2, preferably of at least 4, comprising
- an inlet for fresh hydrogen gas ([M]),
- an outlet of N₂O containing gas ([N]) in the upper region of the hydrogenation reactor ([A]),
- a reaction mixture outlet ([O]) located in the lower region of the hydrogenation reactor ([A]),
- a downward-directed gas-suspension ejector ([F]) in the upper region of the hydrogenation reactor ([A]), wherein the downward directed gas-suspension ejector ([F]) comprises a reaction mixture inlet ([G]), a motive fluid nozzle ([H]), a suction chamber ([I]), a throat ([K]), a divergent outlet diffuser ([L]), and optionally, a convergent inlet nozzle ([J]),
- optionally, a diverter ([P]) arranged below the ejector in the lower region of the hydrogenation reactor ([A]), and
- optionally, a circulation tube ([Q]) arranged below the ejector in the lower region of the reactor ([A]),
- optionally, an inlet of nitrate containing buffered aqueous phosphoric acid containing solution ([T]);
(b) an external circulation loop ([B]), comprising
- a water-cooled indirect heat exchanger ([C]),
- a circulation pump ([D]),
- a filtration device ([E]) comprising an outlet of hydroxylamine-containing buffered aqueous phosphoric acid-containing solution ([R]) inserted into the external circulation loop ([B]) or a line ([Y]) branching off from the external circulation loop ([B]) connecting the external circulation loop ([B]) to a filtration device ([E]) comprising an outlet of hydroxylamine-containing buffered aqueous phosphoric acid containing solution ([R]); and
- optionally an inlet of nitrate-containing buffered aqueous phosphoric acid-containing solution ([T]), and
wherein the external circulation loop ([B]) connects the reaction mixture outlet ([O]) of the hydrogenation reactor ([A]) with the reaction mixture inlet ([G]) of gas-suspension ejector ([F]).

10. The loop venturi reaction device ([X]) of claim 9, wherein the exit velocity of the reaction mixture leaving the liquid nozzle ([H]) is at least 10 m/s, preferably more than 20 m/s, and wherein hydrogen-containing gas is sucked from the suction chamber ([I]).

11. The loop venturi reaction device ([X]) of any of the preceding claims, wherein the filtration device ([E]) is inserted into the external circulation loop ([B]) and is a cross-flow filtration unit.

12. The loop venturi reaction device ([X]) of any of the preceding claims, wherein the water-cooled indirect heat exchanger ([C]) is selected from a shell and tube type heat exchanger or a plate type heat exchanger, preferably a shell and tube type heat exchanger.

13. The loop venturi reaction device of any of the preceding claims, wherein the walls of the loop venturi reaction device ([X]) comprise steel and the steel is selected from the group consisting of:
- quench annealed steel A comprising 0 to 0.08 wt% C, 0 to 2.0 wt% Mn, 0 to 2.0 wt% Si, 0 to 0.045 wt% P, 0 to 0.03 wt% S, 17 to 21 wt% Cr, 0 to 0.03 wt% Mo, 8.0 to 13 wt% Ni, the remainder being Fe and unavoidable impurities;
- low carbon steel B comprising 0 - 0.03 wt% C, 0 to 2.0 wt% Mn, 0 to 1.0 wt% Si, 0 to 0.045 wt% P, 0 to 0.03 wt% S, 17 to 21 wt% Cr, 0 to 0.03 wt% Mo, 8.0 to 13.0 wt% Ni, the remainder being Fe and unavoidable impurities;
- stabilized Steel C comprising 0 to 0.08 wt% C, 0 to 2.0 wt% Mn, 0 to 2.0 wt% Si, 0 to 0.045 wt% P, 0 to 0.04 wt% S, 17 to 21 wt% Cr, 0 to 0.03 wt% Mo, 9.0 to 13.0 wt% Ni and either Ti (minimum: 5 times wt% C (carbon) to maximum: 0.8 wt%) or Nb + Ta (minimum: 8 times wt% C (carbon) to maximum: 1.1 wt%), the remainder being Fe and unavoidable impurities.

14. The loop venturi reaction device ([X]) according to any of the preceding claims, wherein the production capacity for hydroxylamine is at least 100 tons per annum, preferably at least 25,000 tons per annum, more preferably at least 50,000 tons per annum, even more preferably at least 75,000 tons per annum and most preferably at least 100,000 tons per annum.

15. Use of a loop venturi reaction device ([X]) according to any one of claims 9 to 14 as stand-alone unit for the production of hydroxylamine or as a part of a chemical plant for the production of oximes, preferably as part of a chemical plant for the production of oximes, more preferably as part of a chemical plant for the production of butanone oxime, cyclopentanone oxime, cyclohexanone oxime and/or cyclodedocecanone oxime, most preferably as part of a chemical plant for the production of cyclohexanone oxime.
